# EUROPEAN PATENT SPECIFICATION

(11) **EP 3 122 871 B1**
(45) Date of publication and mention of the grant of the patent: **02.09.2020**
(21) Application number: 15769922.4
(22) Date of filing: 25.03.2015
(51) Int. Cl.: C12N 5/02, C12N 5/078, C07K 14/745, C07K 14/52, C07K 14/54, C07K 14/555, C07K 14/78, A61L 27/38, A61L 27/54

(54) **EXTRACELLULAR MATRIX GRAFTS LOADED WITH EXOGENOUS FACTORS**
EXTRAZELLULÄRE MATRIXGRAFTS MIT EXOGENEN FAKTOREN
GREFFONS DE MATRICE EXTRACELLULAIRE CHARGÉS AVEC DES FACTEURS EXOGÈNES

(30) Priority: 25.03.2014 US 201461970344 P; 27.03.2014 US 201461971504 P
(43) Date of publication of application: 01.02.2017
(73) Proprietor: Cook Biotech Incorporated, West Lafayette, IN 47906 (US)
(72) Inventor: HILES, Michael, C., Lafayette, IN 47905 (US); RYAN, Christopher, T., Indianapolis, IN 46208 (US)
(74) Representative: Stafford, Jonathan Alan Lewis
(86) International application number: PCT/US2015/022569
(87) International publication number: WO 2015/148723

(56) References cited:
- WO-A1-95/00632
- WO-A2-2007/011644
- US-A1- 2008 274 184
- US-A1- 2008 299 171
- CHEN, FA-MING ET AL.: 'Toward delivery of multiple growth factors in tissue engineering' BIOMATERIALS vol. 31, no. ISSUE, 2010, pages 6279 - 6308, XP027474530
- HEMEDA, HATIM ET AL.: 'Heparin concentration is critical for cell culture with human platelet lysate.' CYTOTHERAPY vol. 15, no. ISSUE, 2013, pages 1174 - 1181, XP055227968
- FEKETE, NATALIE ET AL.: 'Platelet lysate from whole blood-derived pooled platelet concentrates and apheresis-derived platelet concentrates for the isolation and expansion of human bone marrow mesenchymal stromal cells: production process, content and identification of active components' CYTOTHERAPY vol. 14, no. ISSUE, 2012, pages 540 - 554, XP055178087
- TAYLOR, C. G. ET AL.: 'Comparison of human platelet lysate and fetal bovine serum for optimal culture conditions of neonatal and adult mesenchymal stem cells' CYTOTHERAPY vol. 16, no. ISSUE, April 2014, pages S84 - S85, XP055358824

## Description

### BACKGROUND

The present invention relates generally to the field of medical or research compositions and in certain aspects to matrices that are loaded with bioactive substances that can be derived from animal blood platelet products, and methods of preparation and use thereof.

The administration of tissue-derived compositions for therapeutic treatment is becoming an increasingly popular treatment modality. Animal platelet lysate compositions such as human platelet lysate (hPL) have emerged as potential therapeutic or in vitro reagent materials. Platelet lysates are known to contain a variety of growth factors. While a variety of products and processes for producing and using platelet lysate compositions have been proposed, additional alternatives and/or improvements are in need.

WO2007011644 describes a composition for reconstruction, replacement or repair of a defect or damage in organ tissue. US2008299171 describes tissue graft constructs that include submucosa and other extracellular matrix materials that incorporate a number of exogenous proteins.

### SUMMARY

In certain aspects, the present disclosure provides unique bioactive-loaded matrix compositions and methods for the preparation of use of such compositions. In accordance with some forms of the disclosure, such compositions comprise a collagenous biomaterial which has been treated with a bioactive fraction of mammalian platelets. Accordingly, in one embodiment, the present disclosure provides a composition comprising a collagenous extracellular matrix material, and a bioactive fraction of mammalian platelets applied to the collagenous extracellular matrix material. In one form, the mammalian platelets are human platelets. The bioactive fraction includes TGF-β, EGF, FGF-basic, PDGF-AA, PDGF-BB, SDF-1α, and VEGF. In some forms, the bioactive fraction has a fibrinogen content of less than about 20,000 ng/ml. The collagenous extracellular matrix material includes retained sulfated glycosaminoglycans (e.g. including heparin) native to a source tissue for the collagenous extracellular matrix material. In this regard, the source tissue can be processed to remove native cells of the tissue to form the collagenous extracellular matrix material as an acellular matrix while retaining the sulfated glycosaminoglycans native to the source tissue, for example in the amounts specified in the present disclosure. The use of collagenous extracellular matrix materials that advantageously non-covalently bind bioactive factors, such as growth factors, from the bioactive fraction of platelets, provides the factors in active form at a site of implant of the collagenous extracellular matrix material. The binding of the bioactive factors can provide resistance to migration of the bioactive factors from the site of implant and/or enhance the local biologic effect of the bioactive factors at the site of implant as compared to the administration of the same amount of the bioactive factors in the absence of the collagenous extracellular matrix material. This, in turn, can provide a number of benefits that may include the capacity to use a lower dose of the bioactive factors while achieving the same biologic effect at the site of implant.

In another embodiment, the disclosure provides a method for preparing a bioactive composition, the method comprising applying a bioactive fraction of mammalian platelets to a collagenous extracellular matrix material. In certain aspects, the method also includes rinsing the collagenous extracellular matrix material after applying the bioactive fraction of mammalian platelets to remove a portion of the applied bioactive fraction from the collagenous extracellular matrix material; and/or packaging the bioactive composition in a sterile container; and/or drying (preferably by lyophilization) the collagenous extracellular matrix material after applying the bioactive fraction of mammalian platelets and potentially prior to packaging the bioactive composition in a sterile container.

In an additional embodiment, provided is a kit for preparing a composition, where the kit includes a collagenous extracellular matrix material (e.g. having any feature or combination of features for a collagenous extracellular matrix material described herein) and a bioactive fraction of mammalian platelets (e.g. having any feature or combination of features for a bioactive fraction of mammalian platelets described herein). The collagenous extracellular matrix material and/or the bioactive fraction of mammalian platelets can be in dried (preferably lyophilized) form, in which case the kit can optionally also include a liquid medium for reconstituting the collagenous extracellular matrix material and/or the bioactive fraction of mammalian platelets. The kit can have a package enclosing the components of the kit. The collagenous extracellular matrix material and the bioactive fraction of mammalian platelets can each be sterilely sealed in its own container, and/or wherein the kit can also include at least one vessel (e.g. a syringe or a tub for mixing or wetting) for combining the collagenous extracellular matrix material and the bioactive fraction of mammalian platelets. In related methods for preparing a composition, the collagenous extracellular matrix material and the bioactive fraction of the mammalian platelets can be removed from packaging of the kit, and combined to form a composition, e.g. using any method for combination described herein.

Disclosed herein, but not encompassed within the scope of the claimed invention, is a method of treating a human patient, the method comprising administering to the patient a composition of, or a composition prepared by a method of, any of the embodiments disclosed herein.

Still further embodiments, as well as features and advantages, will be apparent to those of ordinary skill in the art from the descriptions herein.

### BRIEF DESCRIPTION OF THE DRAWINGS

Figure 1 illustrates a method for preparing a liquid bioactive fraction of a human platelet concentrate composition.
Figure 2 is a perspective view of one embodiment of a liquid bioactive fraction of the present disclosure in a sterile package.
Figure 3a is a chart representing the amount of VEGF extracted from the biomaterials tested in Example 2.
Figure 3b is a chart representing the percentage of VEGF present in 1ml HPL extracted from the biomaterials tested in Example 2.
Figure 4a is a chart representing the amount of TGF-β extracted from the biomaterials tested in Example 2.
Figure 4b is a chart representing the percentage of TGF-β present in 1ml HPL extracted from the biomaterials tested in Example 2.
Figure 5a is a chart representing the amount of PDGF-BB extracted from the biomaterials tested in Example 2.
Figure 5b is a chart representing the percentage of PDGF-BB present in 1ml HPL extracted from the biomaterials tested in Example 2.
Figure 6a is a chart representing the MTT absorbance of the samples prepared using the biomaterials tested in Example 2.
Figure 6b is a chart representing the MTT absorbance of the samples prepared using the biomaterials tested in Example 2, as a percentage of the absorbance of a full media sample.

### DETAILED DESCRIPTION

For the purpose of promoting an understanding of the principles of the invention, reference will now be made to certain embodiments and specific language will be used to describe the same. It will nevertheless be understood that no limitation of the scope of the invention is thereby intended. Any alterations and further modifications in the described embodiments, and any further applications of the principles of the invention as described herein are contemplated as would normally occur to one skilled in the art to which the invention relates.

The present disclosure relates *inter alia* to bioactive compositions, methods of making bioactive compositions, and methods of treating a patient using such bioactive compositions. The bioactive composition of the present disclosure comprises a collagenous biomaterial and a bioactive fraction of mammalian platelets applied to the collagenous biomaterial. The collagenous biomaterial comprises collagenous extracellular matrix (ECM) tissue material. In some forms the bioactive fraction of mammalian platelets comprises a platelet lysate composition. In certain embodiments the platelet lysate composition comprises a human platelet lysate (hPL) composition.

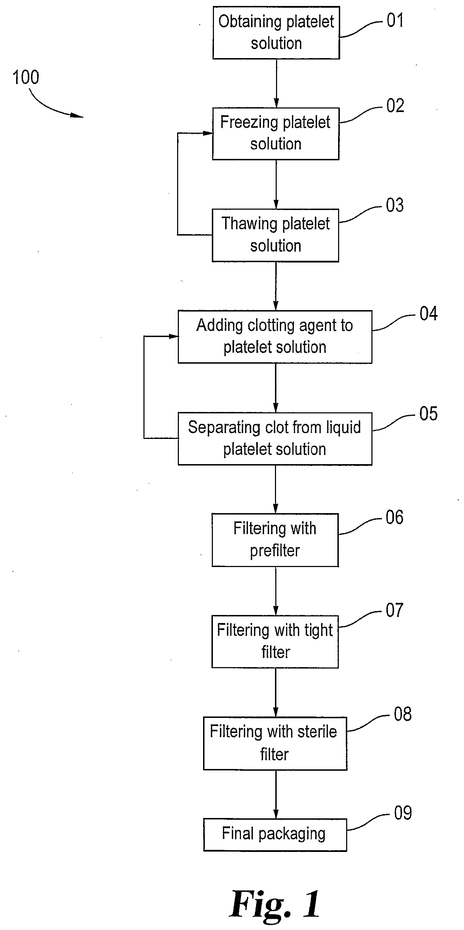

The bioactive fraction of mammalian platelets for use herein can be prepared in any suitable manner. Turning now to FIG. 1, shown is one illustrative method **100** for preparing a bioactive fraction from a platelet concentrate composition. The method includes the steps of: obtaining a platelet concentrate **01,** freezing the platelet concentrate **02,** thawing the platelet concentrate **03,** adding clotting agent to the platelet concentrate **04,** separating clot solids from a liquid **05,** filtering the liquid with a first depth filter **06,** filtering the liquid with a second depth filter **07,** filtering the liquid with a sterile filter **08,** and packaging the liquid **09.** The discussions below in some respects expand upon options for each of these depicted general steps; however, it will be understood that not all of the depicted general steps are required for all embodiments herein, and that novel methods that include features corresponding to one, some, or all of the depicted steps are contemplated as embodiments herein.

Platelet concentrate compositions used as source material for the disclosed methods and bioactive fractions may be obtained in any suitable way. As used herein, the term platelet concentrate refers to a liquid composition containing platelets that have been concentrated from a blood source. The blood source is preferably human blood, such as whole human peripheral blood, although in other embodiments a non-human mammal blood may be used as a source, for example including canine, feline or equine blood. The platelet concentrate preferably includes both platelets and plasma proteins, and may be provided by platelet units obtained from whole peripheral blood of human donors by apheresis. It is envisioned that whole blood from humans or other species, for example mammalian species including those identified above, may also be used as a source for platelet concentrates to be processed as described herein. In certain embodiments, platelet and/or blood units from different humans (or different animals of the same species) can be pooled at some point during processing to obtain the bioactive fraction. In typical practice today, each human donor apheresed platelet unit has a volume of about 100 to about 500 mL, more typically about 100 to 400 mL, and contains about 100 to 500 x 10⁹ platelets along with plasma isolated with the platelets during the apheresis procedure. Donated human apheresis platelet units have a relatively brief shelf life for use at health care facilities, typically about five days. Platelet units used in methods herein can be recently expired human apheresis platelet units obtained from health care facilities, and can optionally be stored frozen at any suitable temperature, for example about -20°C, prior to use to prepare a bioactive fraction as described herein.

In preparing the bioactive fraction, the contents of the platelets can be released by a suitable method. In some modes, the platelets are lysed by subjecting them to at least one freeze-thaw cycle to release the platelet contents, and optionally multiple freeze-thaw cycles (e.g. 2 or 3 freeze-thaw cycles). In use of a freeze-thaw cycle, the platelet concentrate can be frozen at any suitable temperature. In some aspects, the platelet concentrate is frozen at a temperature between about -10°C and about -80°C. In specific preferred embodiments, the platelet concentrate is frozen at about -20°C. To lyse the platelets, the frozen platelet concentrate is thawed, for example in a 37 °C water bath or by other effective means, to form a "raw" platelet lysate composition. The raw platelet lysate contains the lysed platelet membranes and growth factors and other substances released from the lysed platelets. When the platelet concentrate being thawed contains plasma along with the platelets, the platelet lysate will also contain plasma, including plasma proteins therein. Other techniques for releasing platelet contents, for example activation with thrombin, may be used in certain aspects herein. However, freeze-thaw or other mechanical techniques for lysing the platelets are considered advantageous in that they do not require the addition of a non-native protein - thrombin - to the platelet concentrate, which addition both increases cost and leads to the presence of at least some of the thrombin in the downstream processed material.

The raw platelet lysate contains multiple growth factors from the platelet concentrate starting material. These can include, for example, transforming growth factor beta 1, epidermal growth factor, basic fibroblast growth factor, platelet derived growth factor AA, platelet derived growth factor BB, stromal cell-derived factor-1 α, and vascular endothelial growth factor.

Transforming growth factor beta 1 (TGF-β1) is a multifunctional peptide that controls proliferation, differentiation, and other functions in many cell types. Epidermal growth factor (EGF) stimulates cellular proliferation, differentiation, and survival. Basic fibroblast growth factor (FGF-b) promotes angiogenesis, and binds to heparin which stimulates a wide variety of cells. Platelet derived growth factor AA (PDGF-AA) is a dimeric glycoprotein which regulates cell growth and division, and promotes angiogenesis. Platelet derived growth factor BB (PDGF-BB) is a dimeric glycoprotein which regulates cell growth and division, and promotes angiogenesis. Stromal cell-derived factor-1 α (SDF-1α) activates leukocytes and promotes angiogenesis. Vascular endothelial growth factor (VEGF) contributes to vasculogenesis and angiogenesis.

In certain embodiments, the raw platelet lysate includes the following growth factors and amounts thereof (based on the volume of original, undiluted platelet concentrate):
about 50,000 to about 150,000 pg/ml TGF-β1, preferably about 70,000 to about 120,000 pg/ml TGF-β1; and/or
about 100 to 600 pg/ml EGF, preferably about 200 to about 600 pg/ml EGF; and/or
about 5 to about 250 pg/ml FGF-b, preferably about 50 to 200 pg/ml FGF-b; and/or
about 500 to about 20,000 pg/ml PDGF-AA, preferably about 5000 to about 15000 pg/ml PDGF-AA; and/or
about 1000 to about 20,000 pg/ml PDGF-BB, preferably about 2000 to about 15000 pg/ml PDGF-BB; and/or
about 400 to 1100 pg/ml SDF-la, preferably about 500 to about 1000 pg/ml SDF-la.; and/or
about 10 to about 800 pg/ml VEGF, preferably about 100 to about 600 pg/ml VEGF.

In preferred forms, the raw platelet lysate also includes one or more components derived from plasma in the platelet concentrate starting material, including for example fibrinogen, globulins, albumen, triglycerides, glucose, sodium, calcium, and/or cholesterol. In preferred forms, the raw platelet lysate includes the following components and amounts:
about 0.5 to 2.5 g/dL globulins, preferably about 1.5 to 2.5 g/dL globulins;
about 2 to 5 g/dL albumin, preferably about 3 to 4 g/dL albumin;
about 100 to 200 mmol/L sodium, preferably about 120 to about 160 mmol/L sodium;
about 40 to 200 mg/dL triglycerides, preferably about 50 to 120 mg/dL triglycerides;
about 150 to 300 mg/dL glucose, preferably about 150 to 250 mg/dL glucose;
about 5 to 12 mg/dL calcium, preferably about 6 to 10 mg/dL calcium; and/or
about 1 to 3.5 million ng/mL fibrinogen, preferably about 1.5 to 2.5 million ng/mL fibrinogen.

The raw platelet lysate can also contain other bioactive substances, for example one or more interleukins, interferons, and/or tumor necrosis factors. These interleukin(s), interferon(s) and/or tumor necrosis factor(s) may include, for example, one, some, or all of interleukin (IL)-1b, IL-6, IL-8, IL-10, IL-13, IL-17, interferon-gamma (IFN-gamma), and tumor necrosis factor-alpha (TNF-alpha).

In certain embodiments herein, the raw platelet lysate is processed to remove particulate matter, for example centrifuged, and sterilized for use as a platelet lysate product. Such sterilization can, for example, include passing the raw platelet lysate depleted of the particulate matter through a sterile filter.

In some embodiments herein, the raw platelet lysate is treated to recover a fraction thereof with a reduced fibrinogen concentration. Fibrinogen may be removed by any suitable technique, including for example by conversion to fibrin resulting in the formation of solid clot material, which can be separated from a liquid bioactive fraction. Such conversion to fibrin can be induced by the addition of a clotting agent. In accordance with some forms of practicing the disclosed methods, a clotting agent, for example a calcium chloride salt, can be added to the raw platelet lysate. Illustratively, a calcium chloride salt can be added to the raw platelet lysate in an amount between about 0.1g and 2g per liter of raw platelet lysate. In preferred embodiments, about 0.4g to about 0.7g of a calcium chloride salt is added per liter of raw platelet lysate. The combined platelet lysate and calcium chloride or other clotting agent may be placed on a shaker or otherwise agitated to ensure thorough mixing of the clotting agent with the concentrate. The resulting mixture is then allowed to form a solid clot material, in certain embodiments for a period of at least about 8 hours, or at least about 12 hours, and typically in the range of about 8 hours to about 36 hours. In preferred forms, at least a predominant amount (over 50%) of the resulting clotted material, and potentially at least 80% or at least 90% of the resulting clotted material, is constituted by a substantially homogenous clot gel. Such a substantially homogenous clot gel can exhibit a consistent gel phase throughout the material, with liquid entrained within a continuous fibrin matrix. These preferred forms of clotted material are distinct from clotted platelet concentrate materials in which a multitude of discrete solid clot particles are suspended in a liquid phase, as would be desirable for a subsequent centrifuge-based separation technique.

After a clot has formed, liquid material can be separated from solid clot material. Any suitable technique may be used for this purpose. In preferred forms, the clotted material is pressed between two or more surfaces to separate clotted solids from liquid. In cases where the clotted material exhibits the form of a substantially homogenous clot gel as discussed herein, such pressing can express the liquid from the gel material while compressing and condensing the fibrin matrix of the gel. Pressing the clotted material can in some forms be conducted in a flexible container such as a plastic bag. The clot gel can be pressed, for example manually by hand or by forced application of an implement, to one region (e.g. end) of the bag or other flexible container and the liquid expressed from the solid fibrin matrix can gather in another region (e.g. end) of the bag or other flexible container. A second bag or other container can be connected to the first bag in which the pressing occurs, either during or after the pressing, and the liquid material can be transferred to the second bag or other container. In other modes, the clot gel can be in a rigid container such as a bucket, and can by pressed by hand or with the forced application of an implement to express the liquid from the solid fibrin matrix and compress and condense the fibrin matrix.

After clotting and separation of the liquid and solid materials of the clotted platelet concentrate, the separated liquid has a reduced concentration of fibrinogen as compared to the raw platelet lysate prior to clotting. In preferred forms, the raw platelet lysate has a fibrinogen content of at least one million ng/mL, typically in the range of about 1,500,000 to 3,500,000 (1.5 to 3.5 million) ng/mL, and after clotting and separation the liquid has a fibrinogen content of less than about 50,000 ng/mL, preferably less than about 20,000 ng/mL, and more preferably less than about 10,000 ng/mL. Illustratively, this separated liquid can have a fibrinogen content in the range of about 500 ng/mL to about 20,000 ng/mL, or about 500 ng/mL to about 10,000 ng/mL. Additionally or alternatively, this separated liquid can contain less than about 5% of the fibrinogen present in the platelet concentrate prior to clotting, preferably less than about 2%, and more preferably less than about 1%. As well, this separated liquid can constitute at least about 70% of the volume of the raw platelet lysate, preferably at least about 75%, and typically in the range of about 75% to about 90%.

The fibrinogen-depleted liquid bioactive fraction recovered after the clotting of the raw platelet lysate and the liquid solid/separation separation contains multiple growth factors from the raw platelet lysate. These can include TGF-β1, EGF, FGF-beta, PDGF-AA, PDGF-BB, SDF-la , and VEGF. In certain embodiments, this fibrinogen-depleted liquid bioactive fraction includes the following growth factors and amounts thereof from the raw platelet lysate:
about 50,000 to about 150,000 pg/ml TGF-β1, preferably about 70,000 to about 120,000 pg/ml TGF- β1;
about 20 to 800 pg/ml EGF, preferably about 400 to about 800 pg/ml EGF;
about 5 to about 250 pg/ml FGF-b, preferably about 50 to 250 pg/ml FGF-b;
about 500 to about 25,000 pg/ml PDGF-AA, preferably about 5000 to about 18000 pg/ml PDGF-AA; and/or
about 1000 to about 25,000 pg/ml PDGF-BB, preferably about 2000 to about 18000 pg/ml PDGF-BB; and/or
about 400 to 1000 pg/ml SDF-la, preferably about 500 to about 900 pg/ml SDF-la.; and/or
about 10 to about 600 pg/ml VEGF, preferably about 150 to about 450 pg/ml VEGF.

In preferred forms, this fibrinogen-depleted liquid bioactive fraction also includes one or more components derived from plasma in the platelet concentrate starting material, including for example globulins, albumen, triglycerides, glucose, sodium, and/or calcium. Where a calcium chloride salt is used to clot the raw platelet lysate, the calcium present in the separated liquid bioactive agent can be from both the lysate and the added calcium salt. In certain embodiments, this separated liquid bioactive fraction includes the following components and amounts from the raw platelet lysate:
about 0.5 to 2.5 g/dL globulins, preferably about 1 to 2 g/dL globulins;
about 2 to 5 g/dL albumin, preferably about 3 to 4 g/dL albumin;
about 100 to 200 mmol/L sodium, preferably about 120 to about 160 mmol/L sodium;
about 40 to 70 mg/dL triglycerides, preferably about 50 to 65 mg/dL triglycerides; and/or
about 150 to 300 mg/dL glucose, preferably about 150 to 250 mg/dL glucose.

As well, where a calcium chloride salt is used as a clotting agent for the raw platelet lysate, this separated liquid bioactive fraction can in some forms include calcium at a level of about 15 to 35 mg/dL, and preferably about 15 to 25 mg/dL.

Certain inventive embodiments herein include filtering the recovered liquid bioactive fraction after the clotting and liquid/solid separation steps, for example to remove suspended solids such as any remaining platelet debris, cellular debris, and clot solids. In preferred modes, such filtering includes processing the liquid bioactive fraction through at least one depth filter, and preferably multiple depth filters, such as two or three depth filters of potentially differing micron ratings. In this regard, as is known and as used herein, a "depth filter" or "depth filtration" refers to a filter to filtration, respectively, that utilizes a porous filtration medium to retain particles throughout the medium, rather than just on the surface of the medium. Further, as is known and as used herein, "nominal micron rating" as applied to a filter means the particle size above which 98% of all suspended solids will be removed throughout the rated capacity of the filter. Certain inventive variants include filtration through at least one depth filter followed by at least one sterile filter. Additional inventive variants include filtration through at least two depth filters followed by at least one sterile filter. In preferred forms, the depth filter or depth filters used have a filter medium with a positive surface charge.

In certain embodiments, first and second depth filters are used in depth filtration of the liquid bioactive fraction. The first depth filter has a nominal micron rating that is larger than that of the second depth filter. In some forms, the first depth filter has a nominal micron rating between about 10 and 0.1 microns. In preferred embodiments, the first depth filter is has a nominal micron rating between 5 and 0.1 microns, even more preferably between about 3 and 0.2 microns. In certain embodiments, the first depth filter has a cellulose membrane and a filter medium comprised of cellulose fibers and an inorganic filter aid, such as diatomaceous earth, with a positive surface charge.

In certain embodiments, the second depth filter has a nominal micron rating less than that of the first depth filter, for example in some forms less than about 0.5 microns. In preferred embodiments, the second depth filter has a nominal micron rating between 0.5 and 0.001 microns, and more preferably between about 0.1 and 0.001 microns. In certain embodiments, the first depth filter has a cellulose membrane and a filter medium comprised of cellulose fibers and an inorganic filter aid, such as diatomaceous earth, with a positive surface charge.

In preferred forms, the liquid bioactive fraction, after the depth filtration or other filtration to remove suspended solids, still contains multiple growth factors from the raw platelet lysate. These can include TGF-β1, EGF, FGF-beta, PDGF-AA, PDGF-BB, SDF-la , and VEGF. In certain embodiments, this filtered liquid bioactive fraction includes the following growth factors and amounts thereof derived from the raw platelet lysate:
about 5000 to about 75,000 pg/ml TGF-β1, preferably about 5000 to about 60,000 pg/ml TGF-β1;
about 20 to 300 pg/ml EGF, preferably about 50 to about 250 pg/ml EGF;
about 5 to about 150 pg/ml FGF-beta, preferably about 30 to 130 pg/ml FGF-b;
about 200 to about 4000 pg/ml PDGF-AA, preferably about 1000 to about 3000 pg/ml PDGF-AA;
about 50 to about 1000 pg/ml PDGF-BB, preferably about 100 to about 500 pg/ml PDGF-BB;
about 100 to 700 pg/ml SDF-la, preferably about 300 to about 600 pg/ml SDF-la.; and/or
about 10 to 400 pg/ml VEGF, preferably about 40 to about 200 pg/ml VEGF.

In preferred forms, this depth filtered or other filtered liquid bioactive fraction also still includes one or more components derived from plasma in the platelet concentrate starting material, including for example globulins, albumen, triglycerides, glucose, sodium, and/or calcium. Again, where a calcium chloride salt is used to clot the raw platelet lysate, the calcium present in the filtered liquid bioactive agent can be from both the lysate and the added calcium salt. In certain embodiments, this filtered bioactive liquid fraction includes the following components and amounts derived from the raw platelet lysate:
about 0.5 to 2.5 g/dL globulins, preferably about 1 to 2 g/dL globulins;
about 2 to 5 g/dL albumin, preferably about 3 to 4 g/dL albumin;
about 100 to 200 mmol/L sodium, preferably about 120 to about 160 mmol/L sodium;
about 50 to 120 mg/dL triglycerides, preferably about 60 to 110 mg/dL triglycerides; and/or
about 150 to 300 mg/dL glucose, preferably about 150 to 250 mg/dL glucose.

As well, where a calcium chloride salt is used as a clotting agent for the raw platelet lysate, this separated bioactive liquid fraction can in some forms include calcium at a level of about 15 to 60 mg/dL, and preferably about 20 to 50 mg/dL.

The bioactive liquid fraction can also include other bioactive substances, for example one or more interleukins, interferons, and/or tumor necrosis factors. These interleukin(s), interferon(s) and/or tumor necrosis factor(s) may include, for example, one, some, or all of interleukin (IL)-1b, IL-6, IL-8, IL-10, IL-13, IL-17, interferon-gamma (IFN-gamma), and tumor necrosis factor-alpha (TNF-alpha).

As noted above, in some embodiments of methods herein, the liquid bioactive fraction is passed through at least one sterile filter, preferably after passage through the depth filter(s) or other filter(s) to remove suspended solids as discussed above. A variety of sterile filters and associated methods are known and can be used. Exemplary contaminants to be removed by the sterile filter(s) include, for example: staphyloccus aureus, pseudomonas aeruginosa, clostridium sporogenes, candida albicans, aspergillus niger, mycoplasma, and/or bacillus subtilis. The sterile filter(s) may be selected to exhibit relatively low protein binding. After sterile filtration, in preferred forms, the sterile filtered liquid bioactive fraction can have the same components as specified above for the depth filtered or other filtered liquid bioactive fraction, and also has levels of those components within the ranges specified above for the depth or other filtered liquid bioactive fraction. It will be understood, however, that some reduction in the levels of some or all of the components may occur during the sterile filtration.

In certain preferred embodiments, a sterile liquid bioactive fraction composition, which can be obtained by the above-described steps of platelet lysis, fibrinogen depletion, and depth or other filtration to remove suspended particulate, includes:
fibrinogen at a level of less than 20,000 ng/mL of the liquid bioactive fraction, for example in the range of about 500 ng/mL to about 20,000 ng/mL;
albumin at a level of at least 2 mg/dL of the liquid bioactive fraction;
globulin at a level of at least 1 g/dL of the liquid bioactive fraction;
TGF-β1 at a level of at least 5000 pg/mL of the liquid bioactive fraction;
EGF at a level of at least 20 pg/mL of the liquid bioactive fraction;
FGF-beta at a level of at least 5 pg/mL of the liquid bioactive fraction;
PDGF-AA at a level of at least 200 pg/mL of the liquid bioactive fraction;
PDGF-BB at a level of at least 50 pg/mL of the liquid bioactive fraction;
SDF-la at a level of at least 100 pg/mL of the liquid bioactive fraction; and
VEGF at a level of at least 10 pg/mL of the liquid bioactive fraction.

In some forms, liquid bioactive fraction compositions of the present disclosure also have the following characteristics:
an endotoxin level of less than about 10 EU/ml;
less than about 25mg/dL of hemoglobin;
about 4 to 6g/dL total protein;
an osmolarity of about 260 to 340 mmol/kg; and/or
a pH between 6.8 and 7.8.

These characteristics can be present in the raw platelet lysate composition (potentially also after solids removal by centrifugation or otherwise and sterilization), the fibrinogen-depleted liquid bioactive fraction recovered after the clotting and liquid/solid separation (and potentially sterilization), or the fibrinogen-depleted liquid bioactive fraction after depth and/or other filtration to remove suspended solids (and potentially sterilization), as described above. Also, because preferred forms of processing do not need to employ detergent as a processing agent, these compositions can be free or essentially free from detergent residues.

In some modes of operation, the procedures utilized to make the fibrinogen-depleted, filtered (e.g. depth-filtered), liquid bioactive fraction composition of the present disclosure result in reductions in the levels of growth factors, interleukins, interferons and/or tumor necrosis factors identified herein. As examples, in certain embodiments, depth or other filtration of the fibrinogen-depleted fraction is conducted to remove suspended solids, and results in:
at least a 20% reduction in the level (e.g. in pg/mL) of one, some or all of TGF-beta-1, EGF, FGF-b, PDGF-AA, PDGF-BB, SDF-la , and VEGF; and/or
at least a 50% reduction in the level (e.g. in pg/mL) of TGF-beta-1; and/or
at least a 30% reduction in the level (e.g. in pg/mL) of EGF; and/or
at least a 20% reduction in the level (e.g. in pg/mL) of FGF-b; and/or
at least a 50% reduction in the level (e.g. in pg/mL) of PDGF-AA; and/or
at least a 50% reduction in the level (e.g. in pg/mL) of PDGF-BB; and/or
at least a 20% reduction in the level (e.g. in pg/mL) of SDF-la; and/or
at least a 30% reduction in the level (e.g. in pg/mL) of VEGF.

In addition or alternatively, depth or other filtration can result in highly significant levels of removal of interleukin-17 (IL-17). In certain embodiments, the level of IL-17 after depth or other filtration to remove particulate, and also potentially in the final, sterilized liquid bioactive fraction product, is less than about 1 picogram/ml, more preferably less than about 0.75 picograms/ml, and even more preferably less than about 0.5 picograms/ml. IL-17 is an inflammatory cytokine that also cascades in triggering the release of other inflammatory cytokines. Preferred products having low levels of IL-17 as identified herein can be put to use with little or no inflammatory activity stemming from the presence of IL-17.

In addition or alternatively, the depth or other filtration of the fibrinogen-depleted fraction to remove suspended solids can result in a liquid bioactive fraction product that has a concentration of PDGF-BB of less than 1000 pg/mL, a concentration of PDGF-AA of less than 3000 pg/mL, a concentration of TGF-β1 of at least 5000 pg/mL, and/or a concentration of VEGF of less than 300 pg/mL. These values can also be present in a sterilized product prepared (e.g. by sterile filtration) after the depth or other filtration to remove suspended solids.

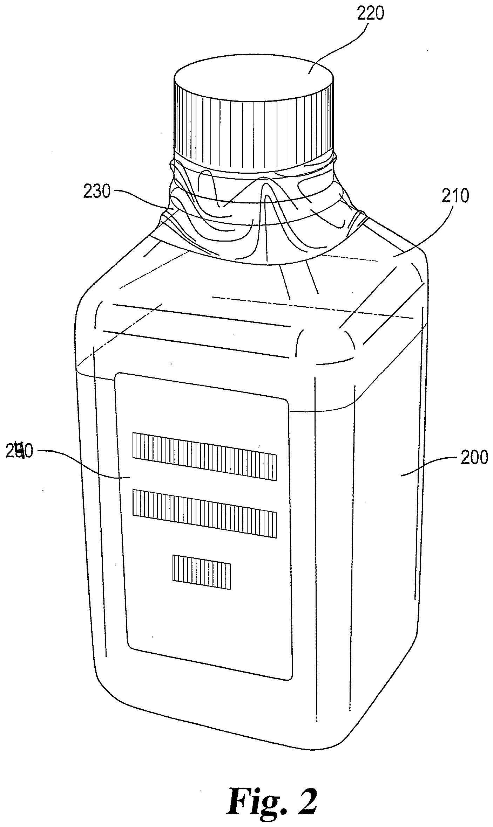

In some forms, liquid bioactive fraction compositions of the present disclosure may be packaged in a sterile package for storage or delivery, for example for later application to the collagenous biomaterial (e.g. at a location of patient care or during another manufacturing step prior to shipping to the location of patient care or other use). The liquid bioactive fraction can be packaged at its full recovered concentration, or it may be diluted with water or an aqueous medium for packaging and later use, for example dilutions to 90% to 10% of the original concentration of the liquid bioactive fraction can be prepared, and such diluted compositions, and their resulting corresponding reductions in the component levels specified herein, form additional embodiments disclosed herein. One embodiment of such packaging is illustrated in FIG. 2. In accordance with some forms of practicing the disclosure, the composition **200** is stored in a sterile media bottle **210.** Sterile media bottles may, for example, have a volume capacity in the range of 50 mL to 5000 mL. As examples, 60mL, 125mL, 250mL, 500mL, 1000mL, or 2000mL bottles may be used. In some forms, cap **220** of sterile media bottle **210** is protected by shrink wrap **230.** In some forms, the bottle is shrink wrapped. In certain embodiments, the bottle is labeled with a finished product label **240.** In some forms, the bottle is placed in a product box with dry ice.

In certain embodiments, the liquid bioactive fraction composition of the present disclosure may be combined with other ingredients to form a cell culture medium, which culture medium can be applied to the collagenous biomaterial. Such a cell culture medium comprises the liquid bioactive fraction of the present disclosure mixed with other nutrients or media for cell culture, including for example those as found in known cell culture media such as Minimum Essential Medium (MEM), or Dulbecco's Modified Eagle Medium (DMEM). A cell culture medium according to the present disclosure is formulated to provide nutrients (e.g. growth factors, etc.) necessary for the growth or maintenance of cells including for example stem and/or progenitor cells, such as mesenchymal stem cells. Such a cell culture medium, in preferred forms, is free from added heparin and is nonetheless free from any clotted material (e.g. as would be evidenced by the appearance of clot particles visible to the naked eye - without magnification).

In other embodiments, the liquid bioactive fraction composition of the present disclosure, or a fraction thereof, can be used as a therapeutic substance in combination with the collagenous biomaterial. For example, the combined bioactive composition can be used as a therapeutic substance for medical treatments, including for treatment of diseased or damaged tissue such as nerve, tendon, bone, muscle, skin (e.g. wound healing), connective, ocular and/or cardiovascular (e.g. heart or aorta) tissue. The liquid bioactive fraction described herein or compositions including it can be delivered to these or other tissues by any suitable means including for example injection (e.g. in combination with the collagenous biomaterial in suspended particulate and/or gel form) or other surgical implantation.

Turning now to a discussion of collagenous extracellular matrix (ECM) materials for use in embodiments of the present disclosure, ECM materials of the invention can be derived from any suitable organ or other tissue source, desirably one containing significant collagenous connective tissue. Human or other animal tissue sources can be used. Non-human animal sources can be warm-blooded vertebrates, including mammals, with bovine, ovine, caprine, and porcine sources being suitable. Suitable ECM materials obtained from these tissue sources can include submucosa, renal capsule membrane, dermal collagen, dura mater, pericardium, fascia lata, serosa, peritoneum or basement membrane layers, including liver basement membrane, which ECM materials or other suitable ECM materials can be in the form of a decellularized collagenous tissue membrane isolated from a mammalian or other animal tissue source. Compositions according to the claimed invention include collagenous extracellular matrix material, wherein the collagenous extracellular matrix material comprises submucosa. Suitable submucosa materials for these purposes include, for instance, intestinal submucosa, including small intestinal submucosa, stomach submucosa, urinary bladder submucosa, and uterine submucosa. It will be well understood that in isolating ECMs that include submucosa, some or all of the original submucosa from the source tissue may be retained, potentially along with materials derived from one or more adjacent tissue layers. Similar principles apply to other collagen-rich layers or other tissues named herein - the recovered ECM material may include some or all of the specified tissue originally present in the source tissue, and/or may remain connected to adjacent tissue(s) in the final processed ECM material.

Processed, naturally-derived ECM materials of the invention will typically include abundant collagen, most commonly being constituted at least about 80% by weight collagen on a dry weight basis. Such naturally-derived ECM materials will for the most part include collagen fibers that are non-randomly oriented, for instance occurring as generally uniaxial or multi-axial but regularly oriented fibers. When processed to retain native bioactive components, the ECM material can retain these components interspersed as solids between, upon and/or within the collagen fibers. Particularly desirable naturally-derived ECM materials for use in the invention will include significant amounts of such interspersed, non-collagenous solids that are readily ascertainable under light microscopic examination. Such non-collagenous solids can constitute a significant percentage of the dry weight of the ECM material in certain inventive embodiments, for example at least about 1%, at least about 3%, and at least about 5% by weight in various embodiments of the invention.

Submucosa-containing or other ECM tissue used in the invention is preferably highly purified, for example, as described in U.S. Patent No. 6,206,931 to Cook et al. or U.S. Patent No. 8,192,763 to Johnson. Thus, preferred ECM material will exhibit an endotoxin level of less than about 12 endotoxin units (EU) per gram, more preferably less than about 5 EU per gram, and most preferably less than about 1 EU per gram. As additional preferences, the submucosa or other ECM material may have a bioburden of less than about 1 colony forming units (CFU) per gram, more preferably less than about 0.5 CFU per gram. Fungus levels are desirably similarly low, for example less than about 1 CFU per gram, more preferably less than about 0.5 CFU per gram. Nucleic acid levels are preferably less than about 5 µg/mg, more preferably less than about 2 µg/mg, and virus levels are preferably less than about 50 plaque forming units (PFU) per gram, more preferably less than about 5 PFU per gram. These and additional properties of submucosa or other ECM tissue taught in U.S. Patent No. 6,206,931 or U.S. Patent No. 8,192,763 may be characteristic of any ECM tissue used in the present invention.

The processed ECM material of the present invention may also exhibit an angiogenic character and thus be effective to induce angiogenesis in a host engrafted with the material, even in the absence of the added bioactive fraction from platelets. In this regard, angiogenesis is the process through which the body makes new blood vessels to generate increased blood supply to tissues. Thus, angiogenic materials, when contacted with host tissues, promote or encourage the formation of new blood vessels. Methods for measuring in vivo angiogenesis in response to biomaterial implantation have been developed. For example, one such method uses a subcutaneous implant model to determine the angiogenic character of a material. See, C. Heeschen et al., Nature Medicine 7 (2001), No. 7, 833-839. When combined with a fluorescence microangiography technique, this model can provide both quantitative and qualitative measures of angiogenesis into biomaterials. C. Johnson et al., Circulation Research 94 (2004), No. 2, 262-268.

It is advantageous to prepare bioremodelable ECM materials for the medical graft materials and methods of the present invention. Such materials that are bioremodelable and promote cellular invasion and ingrowth provide particular advantage. Bioremodelable materials may be used in this context to promote cellular growth within the site in which a medical graft material of the invention is implanted.

As noted above, the processed submucosal (submucosa-containing) ECM material and any other ECM material may retain any of a variety of growth factors or other beneficial bioactive components native to the source tissue. For example, the submucosa or other ECM can include one or more native growth factors such as basic fibroblast growth factor (FGF-2), transforming growth factor beta (TGF-beta), epidermal growth factor (EGF), connective tissue growth factor (CTGF), vascular endothelial growth factor (VEGF) and/or platelet derived growth factor (PDGF). As well, submucosa or other ECM used in the invention may include other native biological materials such as proteoglycans, glycosaminoglycans (GAG), and/or sulfated glycosaminoglycans (sGAG), such as heparin, heparan sulfate, or hyaluronic acid, fibronectin and the like. Thus, generally speaking, the processed ECM material can include at least one native bioactive component that induces, directly or indirectly, a cellular response such as a change in cell morphology, proliferation, growth, protein or gene expression. The collagenous ECM material used in forming bioactive compositions of the present invention includes retained sulfated glycosaminoglycans (e.g. including heparin) native to a source tissue for the collagenous extracellular matrix material at a level of at least about 500 µg per gram (dry weight) of the collagenous ECM material, or at least about 1000 µg per gram (dry weight) of the collagenous ECM material. Additionally, the collagenous ECM material can include fibronectin native to a source tissue for the collagenous ECM material. Such native sGAG-containing and/or native fibronectin-containing collagenous ECM materials can serve as preferred, highly effective matrices for loading and retention of exogenous bioactive factors provided by the bioactive fraction of platelets, including for example exogenous growth factors. It has been discovered that amounts of the exogenous growth factors derived from platelets, including for example VEGF, TGF-β and PDGF-BB, effectively noncovalently bind to the retained native sGAGs and optionally retained native fibronectin and/or to other native components of collagenous ECM materials that have been processed to retain native bioactivity. This makes such collagenous ECM materials especially desirable for use in conjunction with bioactive fractions of platelets and can benefit local biologic effect of bioactive factors provided by the platelets by providing resistance to migration of the factors from the implant site. Such local biologic effects can include for example angiogenesis, cellular proliferation and/or cellular recruitment to the implant site.

In certain preferred embodiments, the processed ECM material will exhibit a component profile wherein the following non-collagen components are present in the stated amounts:

| Component | Preferred Range | More Preferred Range |
|---|---|---|
| Lipid: | less than 5% | less than 3% |
| FGF-2: | greater than 2 ng/g | greater than 5 ng/g |
| IgA: | less than 5 µg/g | less than 1 µg/g |
| HA: | greater than 50 µg/g | greater than 100 µg/g |
| sGAG: | greater than 1000 µg/g | greater than 2000 µg/g |

Visible nuclei less than 200 per 0.263 mm2 less than 100 per 0.263 mm2
Further, in addition to the retention of native bioactive components and the application of exogenous bioactive substances in the bioactive fraction of platelets, non-native bioactive components such as those synthetically produced by recombinant technology or other methods, may be incorporated into the submucosal or other ECM material. These non-native bioactive components may be naturally-derived or recombinantly produced proteins that correspond to those natively occurring in the ECM tissue, but perhaps of a different species (e.g. human proteins applied to collagenous ECMs from other animals, such as pigs). The non-native bioactive components may also be drug substances. Illustrative drug substances that may be incorporated into and/or onto the ECM materials used in the invention include, for example, antibiotics, thrombus-promoting substances such as blood clotting factors, e.g. thrombin, fibrinogen, and the like. These substances may be applied to the ECM material as a premanufactured step, immediately prior to the procedure (e.g. by soaking the material in a solution containing a suitable antibiotic such as cefazolin), or during or after engraftment of the material in the patient.

The bioactive fraction of platelets and/or any other bioactive component can be applied to a submucosa or other collagenous ECM tissue by any suitable means. Suitable means include, for example, spraying, impregnating, dipping, etc. Also, the bioactive fraction of platelets may be applied to the collagenous ECM material at any suitable time, including for example before implantation (e.g. during a manufacturing step or at the point of care) or after implantation. Illustratively, in certain modes, the collagenous ECM material may first be provided at an implant site, and the bioactive fraction of platelets can thereafter be applied to the collagenous ECM material; or the bioactive fraction can be provided at an implant site, and the ECM material can thereafter be provided to the site so as to contact the bioactive fraction and ECM material. In any or all of these modes, in preferred embodiments the ECM will noncovalently bind amounts of bioactive factors from the bioactive fraction, for example through specific binding to native non-collagenous bioactive components of the collagenous ECM as discussed herein. This in turn may aid in retaining the amounts of bioactive factors at the implant site for a duration longer than if the ECM material had not been implanted and/or enhance the local biological effect imparted by the bioactive factors.

In certain embodiments, the collagenous ECM material will beneficially carry a significant load of bioactive factors from the bioactive fraction of platelets. In preferred aspects, the bioactive composition includes VEGF, TGF-β and/or PDGF-BB of the bioactive fraction carried by the collagenous ECM material. In this regard, the VEGF of the bioactive fraction can be present at a level of at least 500, at least 1000, or at least 2000 picograms per milligram of the collagenous extracellular matrix material on a dry weight basis, this value being in the range of 500 to 5000 picograms per milligram or in the range of 1000 to 3000 picograms per milligram in some embodiments; and/or the TGF-β is present at a level of at least 50000, at least 100000, or at least 200000 picograms per milligram of the collagenous extracellular matrix material on a dry weight basis, this value being in the range of 50000 to 500000 or in the range of 100000 to 500000 picograms per milligram in some embodiments; and/or the PDGF-BB is present at a level of at least 5000, at least 7000, at least 8000, or at least 9000 picograms per milligram of the collagenous extracellular matrix material on a dry weight basis, with this value being in the range of 5000 to 15000 picograms per milligram or in the range of 7000 to 15000 picograms per milligram in some embodiments. It will be understood that these loadings of growth factors are not required in all embodiments disclosed herein, and that higher or lower loadings may be provided in other forms.

Submucosal or other ECM tissue of the invention preferably exhibits an endotoxin level of less than about 12 endotoxin units (EU) per gram, more preferably less than about 5 EU per gram, and most preferably less than about 1 EU per gram. As additional preferences, the submucosa or other ECM material may have a bioburden of less than about 1 colony forming units (CFU) per gram, more preferably less than about 0.5 CFU per gram. Fungus levels are desirably similarly low, for example less than about 1 CFU per gram, more preferably less than about 0.5 CFU per gram. Nucleic acid levels are preferably less than about 2 µg/mg, more preferably less than about 1 µg/mg, and virus levels are preferably less than about 50 plaque forming units (PFU) per gram, more preferably less than about 5 PFU per gram.

In certain embodiments, endotoxin levels can be considered in relation to the surface area of one or more isolated, single sheets of an ECM material. In such instances, a sheet of ECM material can exhibit an endotoxin level of less than about 0.25 EU/cm². In preferred embodiments, a sheet of ECM material exhibits an endotoxin level of less than about 0.2 EU/cm², less than about 0.1 EU/cm², and even less than about 0.05/cm². In a most preferred embodiment, a sheet of ECM material exhibits an endotoxin level of less than about 0.025 EU/cm². Multilayer ECM structures including a plurality of bonded or otherwise coupled sheets of ECM material can exhibit similar endotoxin levels based on the surface area of the overall multilayer structure.

The collagenous ECM material of the invention, with or without the applied bioactive fraction of platelets or other applied substances, can be packaged or otherwise stored in a dehydrated or hydrated state. Dehydration of a medical graft material of the invention can be achieved by any means known in the art. Preferably, dehydration is accomplished by either lyophilization or vacuum pressing, although other techniques, for example air drying, can also be used. When stored in a dry state, it will often be desirable to rehydrate the processed ECM material prior to use. In this regard, any suitable wetting medium can be used to rehydrate the medical material, including as examples water or buffered saline solutions.

In certain embodiments, the collagenous ECM material can be crosslinked. Increasing the amount (or number) of crosslinkages within the material and/or between two or more layers of the material can be used to enhance its strength. However, crosslinkages within the medical graft material may also affect its bioremodelability or other bioactive characteristics. Consequently, in certain embodiments, a bioremodelable ECM material will be provided that substantially retains its native level of crosslinking, or the amount and/or type of added crosslinks within the ECM material can be judiciously selected to retain the desired level of bioremodelability or other bioactive characteristic.

For use in the present invention, any introduced crosslinking of the processed ECM material may be achieved by photo-crosslinking techniques, or by the application of a crosslinking agent, such as by chemical crosslinkers, or by protein crosslinking induced by dehydration or other means. Chemical crosslinkers that may be used include for example aldehydes such as glutaraldehydes, diimides such as carbodiimides, e.g., 1-ethyl-3-(3-dimethylaminopropyl)carbodiimide hydrochloride, ribose or other sugars, acyl-azide, sulfo-N-hydroxysuccinamide, or polyepoxide compounds, including for example polyglycidyl ethers such as ethyleneglycol diglycidyl ether, available under the trade name DENACOL EX810 from Nagese Chemical Co., Osaka, Japan, and glycerol polyglycerol ether available under the trade name DENACOL EX 313 also from Nagese Chemical Co. Typically, when used, polyglycerol ethers or other polyepoxide compounds will have from 2 to about 10 epoxide groups per molecule. Preferably, a medical graft material is crosslinked with a crosslinking agent comprising transglutaminase.

Collagenous ECM materials used in the invention can be provided in a variety of physical forms to suit a variety of medical, research or other applications. For example, a collagenous ECM material can be provided as one or more sheets, a paste, a foam, a non-gelled aqueous solution, a powder, or a gel. Combinations of these forms are also contemplated. In this regard, the configuration of the ECM material may be attained before or after the ECM material has been processed as described herein or combined with the bioactive fraction of platelets. Further, an ECM composite material can be manufactured in larger, bulk dimensions, and then divided into smaller products. Moreover, the ECM material may provided in a naturally-derived layer form, or may itself be a manufactured article, such as a sponge or cast sheet, prepared from a naturally-derived ECM material.

Bioactive compositions of the invention including the collagenous ECM material and the bioactive fraction of platelets may be used in a wide variety of medical (including veterinary) applications. Examples include the repair or reconstruction of tissue, such as nervous tissue, dermal tissue such as in wound healing, e.g. application to external dermal wounds, including but not limited to ulcers (e.g. diabetic or other chronic ulcers), cardiovascular tissue (including vascular tissue and cardiac tissue), pericardial tissue, muscle tissue, ocular tissue, periodontal tissue, bone, connective tissue such as tendons or ligaments, in the treatment of gastrointestinal fistulae (e.g. processed into the form of a plug to occlude at least the primary opening of a fistula such as an anorectal, rectovaginal, or enterocutaneous fistula), and others.

In one embodiment, the ECM material is provided as a fluidized composition, for instance using techniques as described in U.S. Patent Nos. 5,275,826 and 5,516,533. In this regard, solutions or suspensions of the ECM material can be prepared by comminuting and/or digesting the material with a protease (e.g. trypsin or pepsin), for a period of time sufficient to solubilize the material and form substantially homogeneous solution. The ECM material is desirably comminuted by tearing, cutting, grinding, shearing or the like. Grinding the material in a frozen or freeze-dried state is advantageous, although good results can be obtained as well by subjecting a suspension of pieces of the material to treatment in a high speed blender and dewatering, if necessary, by centrifuging and decanting excess waste. The comminuted material can be dried, for example freeze dried, to form a powder. Thereafter, if desired, the powder can be hydrated, that is, combined with water or buffered saline and optionally other pharmaceutically acceptable excipients, to form a fluid tissue graft composition, e.g. having a viscosity of about 2 to about 300,000 cps at 25 °C. The higher viscosity graft compositions can have a gel or paste consistency. In these forms, the bioactive fraction of platelets can be combined with the fluidized ECM composition at any suitable point, including for example prior to drying a comminuted and/or gel form ECM material to form a powder, and/or as or as a part of a rehydration medium for an ECM powder material.

A fluidized bioactive composition including the ECM material and the bioactive fraction of platelets can be used as an injectable graft for tissues, for example, bone or soft tissues, in need of repair or augmentation most typically to correct trauma or disease-induced tissue defects. The present fluidized compositions are also used advantageously as a filler for implant constructs comprising, for example, one or more sheets of a collagenous ECM material formed into sealed (sutured) pouches for use in cosmetic or trauma-treating surgical procedures.

In one illustrative particulate preparation, a larger ECM material prepared as described herein is reduced to small pieces (e.g. by cutting) which are charged to a flat bottom stainless steel container. Liquid nitrogen is introduced into the container to freeze the specimens, which are then comminuted while in the frozen state to form a coarse powder. Such processing can be carried out, for example, with a manual arbor press with a cylindrical brass ingot placed on top of the frozen specimens. The ingot serves as an interface between the specimens and the arbor of the press. Liquid nitrogen can be added periodically to the specimens to keep them frozen.

Other methods for comminuting ECM material specimens can be utilized to produce a powder or other particulate material usable in accordance with the present invention. For example, ECM material specimens can be freeze-dried and then ground using a manual arbor press or other grinding means. Alternatively, ECM material can be processed in a high shear blender to produce, upon dewatering and drying, a particulate material.

Further grinding of the ECM material particulate using a prechilled mortar and pestle can be used to produce consistent, more finely divided product. Again, liquid nitrogen can be used as needed to maintain solid frozen particles during final grinding.

To prepare another fluidized ECM material, an ECM material powder can be sifted through a wire mesh, collected, and subjected to proteolytic digestion to form a substantially homogeneous solution. For example, the powder can be digested with 1 mg/ml of pepsin (Sigma Chemical Co., St. Louis Mo.) and 0.1 M acetic acid, adjusted to pH 2.5 with HCl, over a 48 hour period at room temperature. After this treatment, the reaction medium can be neutralized with sodium hydroxide (NaOH) to inactivate the peptic activity. The solubilized ECM material can then be concentrated by salt precipitation of the solution and separated for further purification and/or freeze drying to form a protease-solubilized collagenous ECM material in powder form. This ECM powder form can also include the bioactive fraction of platelets, for example combined prior to or after drying the ECM material to form a powder.

Fluidized compositions of this invention find wide application in tissue replacement, augmentation, and/or repair. The fluidized compositions can be used to induce regrowth of natural connective tissue or bone in an area of an existent defect. By injecting an effective amount of a fluidized composition into the locale of a tissue defect or a wound in need of healing, one can readily take advantage of the bioactive properties of the composition.

In orthopedic applications, a bioactive composition of the invention including the collagenous biomaterial and the bioactive fraction of platelets can be used to repair bone tissue, for instance using the general techniques described in U.S. Pat. No. 5,641,518. Thus, a powder or other particulate form of the bioactive composition can be implanted into a damaged or diseased bone region for repair. The particulate composition can be used alone, or in combination with one or more additional bioactive agents such as physiologically compatible minerals, growth factors, antibiotics, chemotherapeutic agents, antigen, antibodies, enzymes and hormones. In certain forms, the particulate-form implant will be compressed into a predetermined, three-dimensional shape, which will be implanted into the bone region and will substantially retain its shape during replacement of the graft with endogenous tissues.

A bioactive composition herein including the collagenous ECM material and bioactive fraction of platelets can also be used as a cell growth substrate, illustratively in sheet, paste or gel form, potentially in combination with nutrients which support the growth of the subject cells, e.g. eukaryotic cells such as mesenchymal, endothelial, fibroblastic, fetal skin, osteosarcoma, or adenocarcinoma cells (see, e.g. International PCT Application Publication No. WO 96/24661). In preferred forms, the substrate composition will support the proliferation and/or differentiation of human and/or other mammalian cells, including stem cells such as mesenchymal stem cells.

A bioactive composition of the invention including the collagenous ECM material and the bioactive fraction of platelets can also be used in body wall repair, including for example in the repair of abdominal wall defects such as hernias, using techniques analogous to those described in Ann. Plast. Surg., 1995, 35:374-380; and J. Surg. Res., 1996, 60:107-114. In such applications, preferred medical graft materials of the invention promote favorable organization, vascularity and consistency in the remodeled tissue. In dermatological applications, a bioactive composition of the invention can be used in the repair of partial or full thickness wounds and in dermal augmentation using general grafting techniques which are known to the art and literature (see, e.g. Annals of Plastic Surgery 1995, 35:381-388). In addition, in the area of burn treatment, it is generally known to provide a dermal substitute onto which cultured epidermal grafts (preferably cultured epidermal autografts, or CEA's) are transplanted. Such cultured grafts have typically involved transplanting keratinocytes and/or fibroblasts onto the dermal substitute. In accordance with the present invention, the bioactive composition including the collagenous ECM material and bioactive fraction of platelets can be used as the dermal substitute, for example in sheet form, and the CEA accordingly transplanted onto the material.

The bioactive compositions of the invention can also be used in tissue grafting in urogenital applications. For instance, the compositions can be used in urinary bladder repair to provide a scaffold for bladder regeneration, using techniques corresponding to those generally described in U.S. Pat. No. 5,645,860; Urology, 1995, 46:396-400; and J. Urology, 1996, 155:2098. In fluidized form, the inventive bioactive compositions can also find use in an endoscopic injection procedure to correct vesicureteral reflux. In such applications, an injection can be made, for instance in the area under the ureteral orifice of a patient, to induce smooth muscle growth and collagen formation at the injection site.

Generally, when configured for use as a tissue graft, the ECM material included in materials of the invention can include one or more sheets of ECM material that can be cut or otherwise configured to a desired size for its end use. The graft material is in many instances sized larger than the tissue defect to which it is applied. Sizing the medical graft material in this way allows for easy attachment to the surrounding tissue.

Once the ECM graft material, including the applied bioactive fraction of platelets, has been placed on, in, or around the defect, the material can be attached to the surrounding tissue using any of several known suitable attachment means. Suitable attachment means include, for example, biocompatible adhesives (e.g., fibrin glue), stapling, suturing, and the like. Preferably, the medical graft material is attached to the surrounding tissue by sutures. There are a variety of synthetic materials currently available in the art for use as sutures. For example, sutures comprising Prolene™, Vicryl™, Mersilene™, Panacryl™, and Monocryl™, are contemplated for use in the invention. Other suture materials will be well known to those skilled in the art. The aforementioned materials therefore serve merely as examples and, consequently, are in no way limiting.

In other areas, bioactive compositions including the collagenous ECM material and bioactive fraction of platelets can be used in neurologic applications, for example in techniques requiring a dural substitute to repair defects due to trauma, tumor resection, or decompressive procedures.

In sheet form, an ECM material of the invention can be comprised of a single layer or multiple layers of material, having the bioactive fraction of platelets applied to at least a portion thereof and potentially all thereof. Thus, in certain embodiments, a single isolated layer of ECM material or a multilaminate ECM construct can be used. Illustrative multilaminate ECM constructs for use in the invention may, for example, have from two to about ten isolated ECM layers laminated together.

Multilaminate ECM constructs for use in the invention can be prepared in any suitable fashion. In this regard, a variety of techniques for laminating ECM layers together can be used. These include, for instance, dehydrothermal bonding under heated, non-heated or lyophilization conditions, using adhesives, glues or other bonding agents, crosslinking with chemical agents or radiation (including UV radiation), or any combination of these with each other or other suitable methods. For additional information as to multilaminate ECM constructs that can be used in the invention, and methods for their preparation, reference may be made for example to U.S. Patent Nos. 5,711,969, 5,755,791, 5,855,619, 5,955,110, 5,968,096, and to U.S. Patent Application Publication No. 20050049638.

Single layer ECM or multilaminate ECM constructs or other biocompatible materials used in the present invention can have or can lack perforations or slits in their structure, and in certain embodiments can have a meshed structure for example as described in U.S. Application Patent Publication No. 20050021141. Such mesh patterned structures can be used to provide an ECM or other implant segment that is highly deformable for use in the present invention.

In additional embodiments, ECM's used in the invention can be subjected to processes that expand the materials. In certain forms, such expanded materials can be formed by the controlled contact of an ECM material with one or more alkaline substances until the material expands, and the isolation of the expanded material. Illustratively, the contacting can be sufficient to expand the ECM material to at least 120% of (i.e. 1.2 times) its original bulk volume, or in some forms to at least about two times its original volume. Thereafter, the expanded material can optionally be isolated from the alkaline medium, e.g. by neutralization and/or rinsing. The collected, expanded material can be used in any suitable manner in the preparation of a medical device. Illustratively, the expanded material can be enriched with bioactive components, dried, and/or molded, etc., in the formation of a graft construct of a desired shape or configuration. In certain embodiments, a medical graft material and/or device formed with the expanded ECM material can be highly compressible (or expandable) such that the material can be compressed for delivery, such as from within the lumen of a cannulated delivery device, and thereafter expand upon deployment from the device so as to become anchored within a patient and/or cause closure of a tract within the patient.

Expanded ECM materials can be formed by the controlled contact of a processed ECM material as described above with an aqueous solution or other medium containing sodium hydroxide. Alkaline treatment of the material can cause changes in the physical structure of the material that in turn cause it to expand. Such changes may include denaturation of the collagen in the material. In certain embodiments, it is preferred to expand the material to at least about three, at least about four, at least about 5, or at least about 6 or even more times its original bulk volume. The magnitude of the expansion is related to several factors, including for instance the concentration or pH of the alkaline medium, exposure time, and temperature used in the treatment of the material to be expanded.

ECM materials that can be processed to make expanded materials can include any of those disclosed herein or other suitable ECM's. Typical such ECM materials will include a network of collagen fibrils having naturally-occurring intramolecular cross links and naturally-occurring intermolecular cross links. Upon expansion processing as described herein, the naturally-occurring intramolecular cross links and naturally-occurring intermolecular cross links can be retained in the processed collagenous matrix material sufficiently to maintain the collagenous matrix material as an intact collagenous sheet material; however, collagen fibrils in the collagenous sheet material can be denatured, and the collagenous sheet material can have an alkaline-processed thickness that is greater than the thickness of the starting material, for example at least 120% of the original thickness, or at least twice the original thickness.

Illustratively, the concentration of the alkaline substance for treatment of the remodelable material can be in the range of about 0.5 to about 2 M, with a concentration of about 1 M being more preferable. Additionally, the pH of the alkaline substance can in certain embodiments range from about 8 to about 14. In preferred aspects, the alkaline substance will have a pH of from about 10 to about 14, and most preferably of from about 12 to about 14.

In addition to concentration and pH, other factors such as temperature and exposure time will contribute to the extent of expansion, as discussed above. In this respect, in certain variants, the exposure of the collagenous material to the alkaline substance is performed at a temperature of about 4 to about 45 °C. In preferred embodiments, the exposure is performed at a temperature of about 25 to about 40 °C, with 37 °C being most preferred. Moreover, the exposure time can range from at least about one minute up to about 5 hours or more. In some embodiments, the exposure time is about 1 to about 2 hours. In a particularly preferred embodiment, the collagenous material is exposed to a 1 M solution of NaOH having a pH of 14 at a temperature of about 37 °C for about 1.5 to 2 hours. Such treatment results in collagen denaturation and a substantial expansion of the remodelable material. Denaturation of the collagen matrix of the material can be observed as a change in the collagen packing characteristics of the material, for example a substantial disruption of a tightly bound collagenous network of the starting material. A non-expanded ECM or other collagenous material can have a tightly bound collagenous network presenting a substantially uniform, continuous surface when viewed by the naked eye or under moderate magnification, e.g. 100x magnification. Conversely, an expanded collagenous material can have a surface that is quite different, in that the surface is not continuous but rather presents collagen strands or bundles in many regions that are separated by substantial gaps in material between the strands or bundles when viewed under the same magnification, e.g. about 100x. Consequently, an expanded collagenous material typically appears more porous than a corresponding non-expanded collagenous material. Moreover, in many instances, the expanded collagenous material can be demonstrated as having increased porosity, e.g. by measuring for an increased permeability to water or other fluid passage as compared to the non-treated starting material. The more foamy and porous structure of an expanded ECM or other collagenous material can allow the material to be cast or otherwise prepared into a variety of sponge or foam shapes for use in the preparation of medical materials and devices. It can further allow for the preparation of constructs that are highly compressible and which expand after compression. Such properties can be useful, for example, when the prepared medical graft material is to be compressed and loaded into a deployment device (e.g. a lumen thereof) for delivery into a patient, and thereafter deployed to expand at the implant site.

After such alkaline treatments, the material can be isolated from the alkaline medium and processed for further use. Illustratively, the collected material can be neutralized and/or rinsed with water to remove the alkalinity from the material, prior to further processing of the material to form a medical graft material of the invention.

Bioactive compositions of the invention also can be used in conjunction with one or more secondary components to construct a variety of medical devices. In certain embodiments, the bioactive composition is affixed to an expandable member, such as a self-expanding or forcibly expandable (e.g. balloon-expandable) stent or a frame. Such devices of the invention can be adapted for deployment within the cardiovascular system, including within an artery or vein. Certain devices are adapted as vascular valves, for example for percutaneous implantation within arteries, or within veins of the legs or feet to treat venous insufficiency.

Prosthetic valve devices made with bioactive compositions of the invention can be implanted into a bodily passage as frameless valve devices or, as noted above, the ECM material (with the applied bioactive fraction of platelets) can be attached to an expandable frame. The bioactive composition can be used to form biocompatible coverings such as sleeves and/or to form leaflets or other valve structures (see, e.g. WO 99/62431 and WO 01/19285). In one mode of forming a valve structure, the bioactive composition in sheet form can be attached to a stent in a fashion whereby it forms one, two, or more leaflets, cusps, pockets or similar structures that resist flow in one direction relative to another. In a specific application of such devices, such devices constructed as vascular valves are implanted to treat venous insufficiencies in humans, for example occurring in the legs.

In accordance with certain inventive variant the present disclosure includes a method of making a bioactive composition comprising applying a bioactive fraction of mammalian platelets to a collagenous extracellular matrix material. The bioactive fraction can be applied in any suitable manner. In some forms, the bioactive fraction is applied by dipping or soaking the collagenous ECM material in, or spraying the collagenous ECM material with, a liquid composition of or including the bioactive fraction.

In certain embodiments the collagenous ECM material is rinsed after the bioactive fraction is applied. In accordance with some modes of practicing the disclosed method the collagenous ECM material is rinsed to remove a portion of the bioactive fraction from the collagenous extracellular matrix material, for example with such rinsing removing at least a portion of one bioactive factor, an potential a portion of each of a plurality of bioactive factors, from the collagenous ECM material. In certain forms, the rinsing will preferentially remove one or some bioactive factor(s) relative to others, resulting in a greater percentage reduction of the level (e.g. in percentage by weight) of the preferentially-removed bioactive factor(s) remaining on the collagenous ECM material relative to the others. In this manner, advantageous compositional profiles of the modified bioactive collagenous ECM material can be achieved.

In some forms the disclosed method also comprises drying the collagenous ECM material. The collagenous ECM material can be dried after application of the bioactive fraction. In certain embodiments, the collagenous ECM material is dried after the material is rinsed, and in others it is dried without rinsing. In some forms the collagenous ECM material, including the applied bioactive fraction of platelets is lyophilized (e.g. without rinsing or after rinsing to remove a portion of the applied bioactive fraction of platelets).

The disclosed method may further include packing the bioactive composition in a sterile container. In some forms the bioactive composition is packaged after drying. It is also envisioned that the bioactive fraction may be separately packaged, for example as illustrated in Fig. 2. In some forms, the bioactive fraction and the collagenous ECM material are separately sterilely packaged so as to be combined prior to use, for example included in a kit including both sterilely packaged components and potentially other components. The kit can, for example, include packaging that contains the bioactive fraction in its own sterile container, and the collagenous ECM material in its own sterile container. One or more mixing or wetting vessels such as syringes or tubs or trays may also be included in the kit, as well as any liquid mediums that may be needed to reconstitute or otherwise wet the collagenous ECM material and/or the bioactive fraction (e.g. when either or both are packaged in a dried form, such as a lyophilized or air dried form). In addition or alternatively, it is contemplated that in use to treat a patient, the collagenous ECM material, the bioactive fraction, or their combination, may be combined with an autologous biological material from the patient to be treated, for example a liquid blood or a liquid blood fraction (e.g. serum) of the patient to be treated, prior to administration to the patient. Such autologous biological material can provide additional bioactive substances, such as autologous growth factors or other autologous proteins, to the administered composition.

For the purpose of promoting further understanding of aspects of the present disclosure and their features and advantages, the following specific examples are provided. It will be understood that these examples are illustrative, and not limiting, of embodiments of the present disclosure.

### EXAMPLES

### Example 1

### Preparation of Human Platelet Lysate Composition

Disease-screened apheresed human platelet units (obtained from peripheral blood) that had just expired after a 5-day shelf life are collected and frozen at -20°C in a freezer until use. A number of the units (e.g. about 10 units) are removed from the freezer and thawed at room temperature, thus lysing the platelets and forming a "raw hPL" composition. The raw hPL from the selected units is pooled into a bag. Calcium chloride is added to the pooled raw hPL at a level of 0.7 grams/L (approximately 6 mM CaCl₂) and then thoroughly mixed with the raw hPL on a shaker at room temperature for 2 hours. After mixing, the CaCl₂-treated raw hPL is allowed to clot overnight at room temperature, during which a firm, substantially homogeneous clotted gel mass forms from the volume of raw hPL.

While remaining closed, the bag containing the gel clot of raw hPL is manually pressed by hand to express liquid from the gel clot. This pressing is thoroughly done, resulting in a solid clot mass at one end of the bag and a separate liquid volume at the other end of the bag, adjacent an outlet spout. The separated liquid represents approximately 75-80% of the volume of the original, pooled raw hPL, and the solid clot material represents the remainder. The liquid is transferred from the bag to a second, refrigerated bag having a volume of 100L. A sufficient number of such thaw-pool-clot-express runs are conducted to fill the refrigerated 100L bag with liquid.

The liquid in the 100L bag is connected aseptically to and processed through a filter train constituted of a first depth filter having a filter medium with a positive surface charge and a nominal micron rating of between 3 and 0.2 microns and a second depth filter having a filter medium with a positive surface charge and a nominal micron rating of between 0.1 and .001 microns. The filtration is conducted with a filtrate flux rate of about 100 liters per square meter of filter surface area per hour ("LMH"). The first depth filter is provided by a Millistack Pod Filter, Grade CO Series HC Depth Filter, and the second depth filter is provided by a Millistack Pod Filter, Grade XO Series HC Depth Filter, both commercially available from Millipore Corporation. Each of these filters has a membrane composed of mixed esters of cellulose and filter media composed of cellulose fibers with an inorganic filter aid (diatomaceous earth). Prior to processing the 100L bag material, the filter train is primed with sterile, distilled water. The hPL liquid exiting the filter train is collected into a second 100L bag.

The second 100L hPL bag is aseptically connected to and pumped through a sterile filter into smaller containers, for example 100 mL or 500 mL jars (e.g. Nalgene jars). This can be done under sterile fill conditions. The jars can be shrink-wrapped to cover their capped ends, and labeled.

An hPL product produced in accordance with this Example has a compositional profile as specified herein and can be used as a supplement to cell culture media without the requirement of adding heparin to prevent clot formation. The addition of this hPL product to a cell culture medium results in an essentially clot-free medium, even without the addition of heparin. The cell culture media so produced exhibit excellent properties in the culture of cells, including but not limited to bone marrow mesenchymal cells, adipocyte stem cells, placenta derived mesenchymal stem cells, and muscle-derived stem or progenitor cells, with relatively high cell counts or percent confluence after a given culture period being obtainable in preferred uses.

### Example 2

### Analysis of Matrix Materials Treated with Human Platelet Lysate

A study was preformed to analyze the ability of different biomaterials to deliver growth factors and cell proliferation capabilities of human platelet lysate. The biomaterials tested were small intestine submucosa (SIS, Cook Biotech Inc. - includes significant retained native bioactive materials of the source tissue including sGAGs, fibronectin), dermis (Strattice®, LifeCell), pericardium (Veritas®, Synovis), and an absorbable synthetic polymer (BioA®, Gore). The submucosa sample comprised an 8-layer SIS construct which had been lyophilized and ethylene oxide sterilized, resulting in a sheet construct for which 1cm² of the sheet weighed approximately 0.024 grams. For each assay, 6 test samples were cut (0.5cm x 2cm strips) from each test material with sterile scissors and put into labeled sterile 1.5ml Eppendorf tubes.

### ELISA Assay

1ml of human platelet lysate (HPL) was added to half of the test sample tubes for each test material for each assay and to three additional empty tubes (HPL control samples) for ELISA testing. 1ml of 1x PBS was added to the other sample tubes which did not receive HPL to be the non-treatment test articles. The tubes were placed on a tube rotator at room temperature for 1 hour. The test articles were gently removed from the tubes with sterile tweezers and placed in new labeled 1.5ml Eppendorf tubes. The previous test article tubes of the ELISA test articles were stored for depletion samples.

To each sample tube (treated and non-treated) 400µl of 1x PBS was added. The samples were ground with a crystal grinder for three 30-second periods. The sample tubes were then centrifuged for 5 minutes at 12 thousand rpm, and then the extracts transferred into new 1.5ml Eppendorf tubes and stored in the refrigerator overnight. The following day, the HPL control samples, depletion samples, and test article extracts were diluted to the appropriate dilution either before or within ELISA test kit protocols per the instructions of the ELISA kit protocol of the Quantikine ELISA tests from R&D Systems: TGF-β (#DB100B), PDGF-BB (DBB00), and VEGF (DVE00). TGF-β dilution = 1:00 (including the activation number). VEGF dilution = none. PDGF-BB dilution = 1:5. The ELISA standards, HPL control samples, depletion samples, treated test samples extracts, and non-treated test sample extracts plated in duplicate according to the plate diagram included as Table 1 below, ELISA run according to kit protocol.

**Table 1**

| | 1 | 2 | 3 | 4 | 5 | 6 | 7 | 8 | 9 | 10 | 11 | 12 |
|---|---|---|---|---|---|---|---|---|---|---|---|---|
| A | 2000 pg/ml control | | HPLa | | SISc Extraction | | Strattice(b) Extract | | Veritas(a) Extract | | BioA(c) Deplete | |
| B | 10000 pg/ml control | | HPLb | | SiSa alone | | Strattice(c) Extract | | Veritas(b) Extract | | BioA(a) Extract | |
| C | 500 pg/ml control | | HPLc | | SISb Alone | | Strsttice(a) Alone | | Verittas(c) Extract | | BioA(b) Extract | |
| D | 250 pg/ml control | | SISa Depletion | | SISc Alone | | Strattice(b) Alone | | Veritas(a) Alone | | BioA(c) Extract | |
| E | 125 pg/ ml control | | SISb Depletion | | Strattice(a) Deplete | | Strattice(c) Alone | | Veritas(b) Alone | | BioA(a) Alone | |
| F | 62,5 pg/ml control | | SISc Depletion | | Strattice(b) Deplete | | Veritast(a) Deplete | | Veritas(c) Alane | | BioA(b) Alone | |
| G | 31,2 pg/ml control | | SISa Extraction | | Strattice(c) Deplete | | Veritas(b) Deplete | | BioA(a) Deplete | | BioA(c) Alone | |
| H | 0 pg/ml control | | SISb Extraction | | Strattice(a) Extract | | Veritas(c) Deplete | | BioA(b) Deplete | | | |

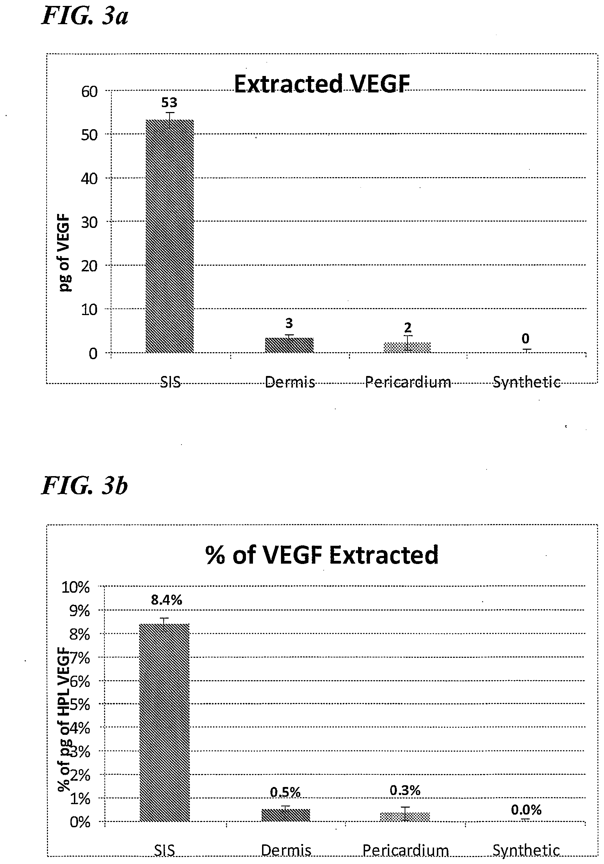

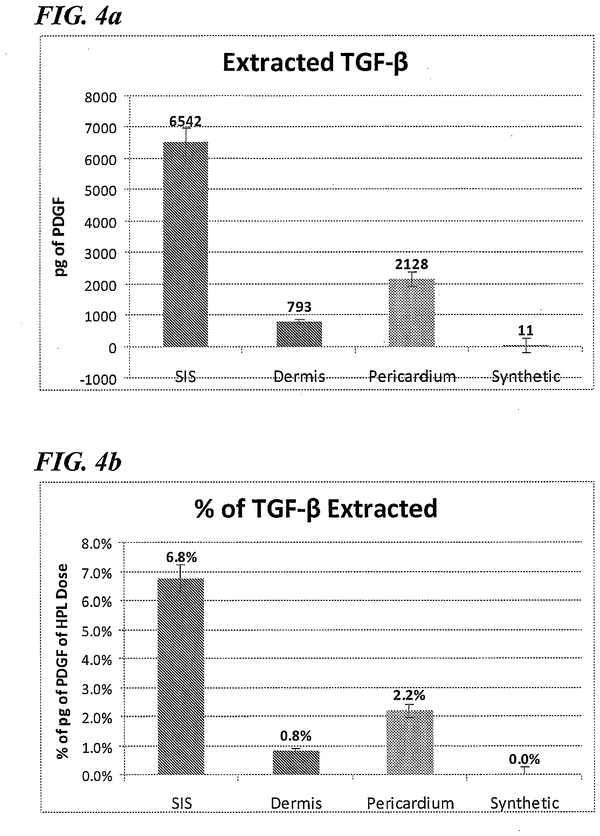

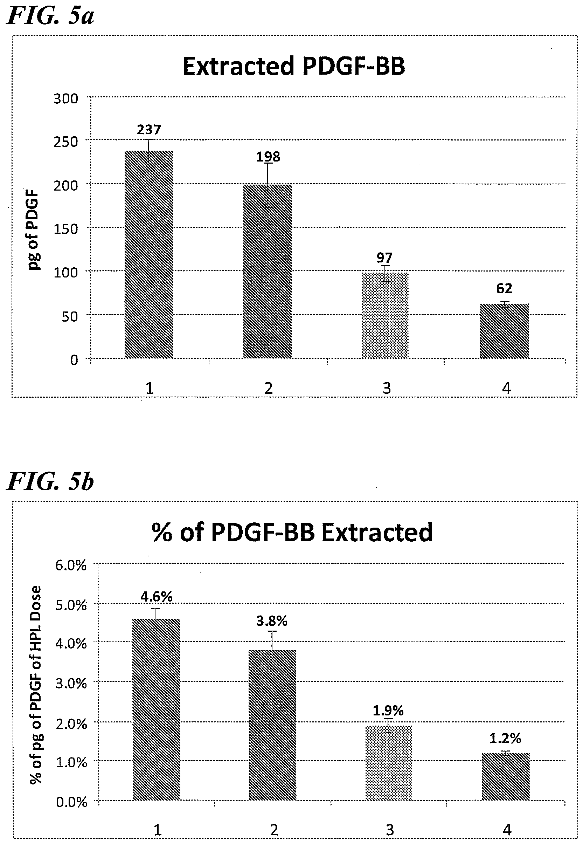

The results of the ELISA assay detailed above are presented in Figures 3a, 3b, 4a, 4b, 5a, and 5b. Figures 3a, 4a, and 5a are charts representing the amount of VEGF, TGF-β, and PDGF-BB extracted from the tested biomaterials as described above. In each test, 1ml HPL was used with 1 cm² of biomaterial, therefore the results indicate pg/ml HPL and pg/cm² of biomaterial. With reference to figure 3a, 53pg of VEGF was extracted from the SIS sample, 3pg from the dermis, 2pg from the pericardium, and 0pg from the synthetic absorbable polymer. With reference to figure 4a, 6542pg of TGF-β was extracted from the SIS sample, 793pg from the dermis, 2128pg from the pericardium, and 11pg from the synthetic absorbable polymer. With reference to figure 5a, 237pg of PDGF-BB was extracted from the SIS sample, 198pg from the dermis, 97pg from the pericardium, and 62pg from the synthetic absorbable polymer.

Figures 3b, 4b, and 5b are charts representing the percentage of the stated growth factor (VEGF, TGF-β, and PDGF-BB respectively) which was bound to the biomaterial. The percentage was calculated by dividing the mean amount (pg) obtained by extraction as detailed above, by the mean amount (pg) detected in 1ml of HPL subjected to the same environmental conditions. With reference to figure 3b, 8.4% of VEGF was extracted from the SIS sample, 0.5% from the dermis, 0.3% from the pericardium, and 0% from the synthetic absorbable polymer. With reference to figure 4b, 6.8% of TGF-β was extracted from the SIS sample, 0.8% from the dermis, 2.2% from the pericardium, and 0% from the synthetic absorbable polymer. With reference to figure 5b, 4.6% of PDGF-BB was extracted from the SIS sample, 3.8% from the dermis, 1.9% from the pericardium, and 1.2% from the synthetic absorbable polymer.

It is envisioned that the amount of growth factor retained by a collagenous ECM material may be optimized by either increasing or decreasing the ratio of ECM material volume to HPL. It is further envisioned that the amount of growth factor retained by a collagenous ECM material may exceed the amount extracted due to strong bonding between the ECM material and the growth factors. For example, ECM components such as fibronectin have been shown to strongly bind growth factors which might prevent them from being extracted.

### MTT Proliferation Assay

An MTT assay (ATCC MTT Cell Proliferation Assay) was performed to measure the viability of a group of cells incubated in either: full media, 5% HPL, or serum-free media. The biomaterial samples were prepared as described above. 1ml of human platelet lysate was added to half of the test sample tubes for each test material for each assay. 1ml of clear SF-dMEM was added to the other sample tubes which did not receive HPL to be the non-treatment test articles. The tubes were placed on a tube rotator at room temperature for 1 hour. The test articles were gently removed from the tubes with sterile tweezers and placed in new labeled 1.5ml Eppendorf tubes.

To each sample tube (treated and non-treated) 1ml of clear SF-dMEM was added. The tubes were incubated on an orbital shaker (∼100rpm) for 24 hours. NIH 3T3 cells were plated (10,000 per well) in full media on a 96-well plate. The plate was incubated overnight at 37°C with 5% Co₂. The first three wells of the last three rows (E1:H3) left without cells for plate absorbance control wells. Using a multichannel pipette, the wells of the 96-well plate were aspirated leaving a minute amount of the media to prevent the cells from drying out. 100µl of serum free media added to each well to rinse out the full media. For each of the control solutions indicated in Table 2 below, three wells were aspirated and 100µl of the appropriate solution add to each of the wells according to the plate diagram included as Table 3 below. For each test and control sample tube 3 wells were aspirated on the plate, the test sample tubes were mixed by pipetting up and down 3 times, and 100µl of each extract was transferred into each of the 3 wells (triplicate wells for each extracts).

**Table 2**

| | |
|---|---|
| Positive Control | Full Media |
| HPL Control | 5% HPL in serum-free media |
| Negative Control | Serum-free media |
| Plate Absorption Control | Serum-free media without cells |

**Table 3**

| | | | | | | | | | | | | |
|---|---|---|---|---|---|---|---|---|---|---|---|---|
| 1 | | 2 | 3 | 4 | 5 | 6 | 7 | 8 | 9 | 10 | 11 | 12 |
| A | Full Media (positive control) | | | Treated SISa | | | Treated Strattice c | | | Untreated Veritas b | | |
| B | 5% HPL in SF-Media (HPL control) | | | Treated SIS b | | | Untreated Strattice a | | | Untreated Veritas c | | |
| C | SF-Media (negative control) | | | Treated SIS c | | | Untreated Strattice b | | | Treated BioA a | | |
| D | | | | Untreated SIS a | | | Untreated Strattice c | | | Treated BioA b | | |
| E | SF- Media w/o cells (plate absorbables) | | | Untreated SIS b | | | Treated Veritas a | | | Treated BioA c | | |
| F | | | | Untreated SIS c | | | Treated Veritas b | | | Untreated BioA a | | |
| G | | | | Treated Strattice a | | | Treated Veritas c | | | Untreated BioA b | | |
| H | | | | Treated Strattice b | | | Untreated Veritas a | | | Untreated BioA c | | |

The plate was incubated for 72 hours at 37°C with 5% CO₂. 10µl of MTT Reagent was added to each well and the plate returned to cell culture incubator for 2-4 hours, during which periodically the cells were viewed under an inverted microscope for the presence of intracellular punctuate purple precipitate. When purple precipitate became clearly visible under the microscope, 100µl of Detergent Reagent from MTT kit add to all wells and swirled gently. Plate was covered and left without agitation for 2-4 hours at room temperature. Absorbance in each well measured at 570nm in a 96-well plate reader. For the true absorbance value, the averages of the triplicate well readings were subtracted from the average values of the plate absorption control (SF-dMEM, no cells).

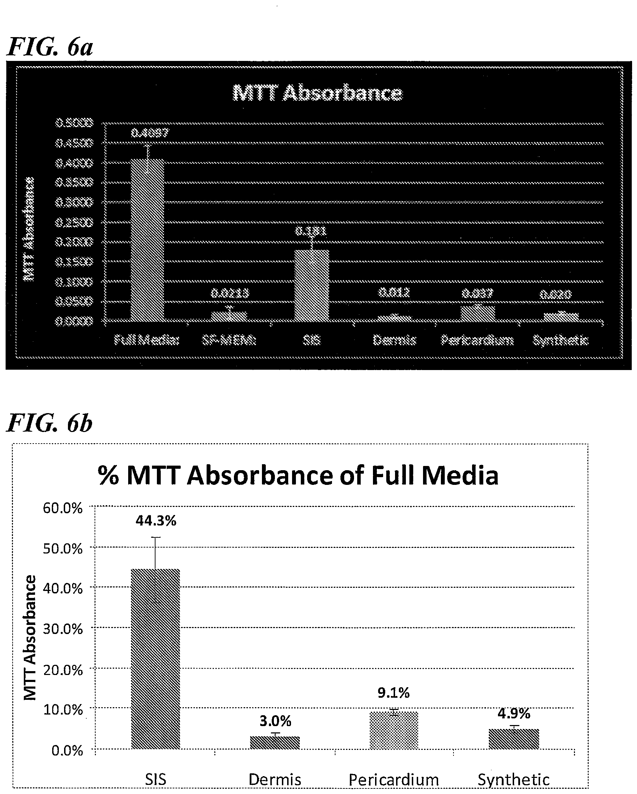

The results of the MTT assay detailed above are presented in Figures 6a and 6b. Metabolic activity is an indicator of how many cells are still alive and functional. Figure 6a is a chart representing the MTT absorbance of samples given full media, serum-free media, SIS, dermis, pericardium, or synthetic absorbable polymer as detailed above. Figure 6b presents the MTT absorbance of the four tested biomaterials as a percentage of the absorbance of the full media sample. The SIS sample demonstrated 0.181 MTT absorbance, 44.4% MTT absorbance of the full media sample, whereas the dermis, pericardium, and synthetic samples performed similarly to serum-free media.

The use of the terms "a" and "an" and "the" and similar referents in the context of describing the invention (especially in the context of the following claims and the above Listing of Embodiments) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Any theory, mechanism of operation, proof, or finding stated herein is meant to further enhance understanding of the present invention, and is not intended to limit the present invention in any way to such theory, mechanism of operation, proof, or finding. While the invention has been illustrated and described in detail in the drawings and foregoing description, the same is to be considered as illustrative and not restrictive in character, it being understood that only selected embodiments have been shown and described.. The scope of protection is defined by the appended claims.

## Claims

1. A composition comprising:
a collagenous extracellular matrix material, wherein the collagenous extracellular matrix material comprises submucosa, and wherein the collagenous extracellular matrix (ECM) material includes retained sulfated glycosaminoglycans native to a source tissue for the collagenous extracellular matrix material at a level of at least about 500 µg per gram of the collagenous ECM material on a dry weight basis; and
a bioactive fraction of mammalian platelets applied to the collagenous extracellular matrix material, wherein the bioactive fraction includes TGF-β1, EGF, FGF-basic, PDGF-AA, PDGF-BB, SDF-la, and VEGF.

2. The composition of claim 1, wherein the mammalian platelets are human platelets.

3. The composition of claim 1 or 2, wherein the collagenous extracellular matrix material is porcine, bovine, ovine or equine extracellular matrix material.

4. The composition of any preceding claim, wherein the bioactive fraction is a bioactive fraction of a human blood-derived platelet concentrate, the platelet concentrate containing human platelets and human plasma, the bioactive fraction comprising native components of the platelet concentrate including fibrinogen, albumin, globulin, TGF-β1, EGF, FGF-basic, PDGF-AA, PDGF-BB, SDF-la , and VEGF.

5. The composition of any preceding claim, wherein the fibrinogen of the bioactive fraction is present at a level of less than 20,000 ng/mL.

6. The composition of any preceding claim, wherein the bioactive fraction also includes at least one of, and preferably each of, IL-lb, IL-6, IL-8, IL-10, IL-13, IL-17, IFN-gamma, and TNF-alpha native to the platelets.

7. The composition of any preceding claim, wherein the bioactive fraction is a liquid bioactive fraction, and wherein the composition includes:
about 0.5 to 2.5 g/dL globulins, preferably about 1 to 2 g/dL globulins;
about 2 to 5 g/dL albumin, preferably about 3 to 4 g/dL albumin;
about 100 to 200 mmol/L sodium, preferably about 120 to about 160 mmol/L sodium;
about 50 to 120 mg/dL triglycerides, preferably about 60 to 110 mg/dL triglycerides; and/or
about 150 to 300 mg/dL glucose, preferably about 150 to 250 mg/dL glucose.

8. The composition of any preceding claim, wherein the bioactive fraction is a liquid bioactive fraction, and wherein the bioactive fraction includes the following components derived from the platelets:
fibrinogen at a level of less than 20,000 ng/ml of the liquid bioactive fraction;
albumin at a level of at least 2 mg/dL of the liquid bioactive fraction;
globulin at a level of at least 1 g/dL of the liquid bioactive fraction;
TGF-β1 at a level of at least 5000 pg/mL of the liquid bioactive fraction;
EGF at a level of at least 20 pg/mL of the liquid bioactive fraction;
FGF-beta at a level of at least 5 pg/mL of the liquid bioactive fraction;
PDGF-AA at a level of at least 200 pg/mL of the liquid bioactive fraction;
PDGF-BB at a level of at least 50 pg/mL of the liquid bioactive fraction;
SDF-1α at a level of at least 100 pg/mL of the liquid bioactive fraction; and
VEGF at a level of at least 10 pg/mL of the liquid bioactive fraction.

9. A method for preparing a bioactive composition, comprising:
applying a bioactive fraction of mammalian platelets to a collagenous extracellular matrix material, wherein the collagenous extracellular matrix material comprises submucosa, wherein the collagenous extracellular matrix (ECM) material includes retained sulfated glycosaminoglycans native to a source tissue for the collagenous extracellular matrix material at a level of at least about 500 µg per gram of the collagenous ECM material on a dry weight basis, and wherein the bioactive fraction includes TGF-β1, EGF, FGF-basic, PDGF-AA, PDGF-BB, SDF-1α, and VEGF.

10. The method of claim 9, wherein the mammalian platelets are human platelets.

11. The method of claim 9 or 10, wherein the collagenous extracellular matrix material is porcine, bovine, ovine or equine extracellular matrix material.

12. The method of any one of claims 9 to 11, wherein the bioactive fraction is a bioactive fraction of a human blood-derived platelet concentrate, the platelet concentrate containing human platelets and human plasma, the bioactive fraction comprising native components of the platelet concentrate including fibrinogen, albumin, globulin, TGF-β1, EGF, FGF-basic, PDGF-AA, PDGF-BB, SDF-1α, and VEGF.

13. The method of any one of claims 9 to 12, wherein the fibrinogen of the bioactive fraction is present at a level of less than 20,000 ng/mL.

14. The method of any one of claims 9 to 13, wherein the bioactive fraction also includes at least one of, and preferably each of, IL-lb, IL-6, IL-8, IL-10, IL-13, IL-17, IFN-gamma, and TNF-alpha native to the platelets.

15. The method of any one of claims 9 to 14, wherein the bioactive fraction is a liquid bioactive fraction, and wherein the bioactive fraction includes the following components derived from the platelets:
fibrinogen at a level of less than 20,000 ng/ml of the liquid bioactive fraction;
albumin at a level of at least 2 mg/dL of the liquid bioactive fraction;
globulin at a level of at least 1 g/dL of the liquid bioactive fraction;
TGF-β1 at a level of at least 5000 pg/mL of the liquid bioactive fraction;
EGF at a level of at least 20 pg/mL of the liquid bioactive fraction;
FGF-beta at a level of at least 5 pg/mL of the liquid bioactive fraction;
PDGF-AA at a level of at least 200 pg/mL of the liquid bioactive fraction;
PDGF-BB at a level of at least 50 pg/mL of the liquid bioactive fraction;
SDF-1α at a level of at least 100 pg/mL of the liquid bioactive fraction; and
VEGF at a level of at least 10 pg/mL of the liquid bioactive fraction.

16. The method of any one of claims 9 to 15, also comprising drying the collagenous extracellular matrix material after said applying and preferably wherein the drying comprises lyophilizing.

17. The method of any of claims 9 to 16, also comprising rinsing the collagenous extracellular matrix after said applying to remove a portion of the bioactive fraction from the collagenous extracellular matrix material.

18. The method of claim 17, wherein the portion includes an amount of at least one growth factor, and preferably a plurality of growth factors.

19. The method of claim 17 or 18, also comprising drying the collagenous extracellular matrix material after said rinsing, and preferably wherein the drying comprises lyophilizing.

20. A composition or method of any preceding claim, wherein the collagenous extracellular matrix material includes collagen in an amount of at least about 80% by weight on a dry weight basis.

21. A composition or a method of any preceding claim, wherein the collagenous extracellular matrix (ECM) material includes retained sulfated glycosaminoglycans native to a source tissue for the collagenous extracellular matrix material at a level of at least about 1000 µg per gram of the collagenous ECM material on a dry weight basis.

22. A composition or a method of any preceding claim, wherein the submucosa is intestinal, urinary bladder or stomach submucosa.

23. A kit for preparing a composition, comprising a collagenous extracellular matrix (ECM) material as defined in any one of claims 1 to 22, and a bioactive fraction of mammalian platelets as defined in any one of claims 1 to 22.

## Patentansprüche

1. Zusammensetzung, Folgendes umfassend:
extrazelluläres Kollagenmatrixmaterial, wobei das extrazelluläre Kollagenmatrixmaterial Submukosa umfasst, und wobei das extrazelluläre Kollagenmatrixmaterial (ECM) zurückgehaltene sulfatierte, native Glykosaminoglykane eines Quellgewebes für das extrazelluläre Kollagenmatrixmaterial mit einem Spiegel von mindestens ungefähr 500 µg pro Gramm des ECM-Kollagenmaterials auf einer Trockengewichtsgrundlage beinhaltet; und
einen bioaktiven Anteil an Säugetier-Blutplättchen, welcher auf das extrazelluläre Kollagenmatrixmaterial aufgetragen ist, wobei der bioaktive Anteil TGF-β1, EGF, FGF-basic, PDGF-AA, PDGF-BB, SDF-1α und VEGF beinhaltet.

2. Zusammensetzung nach Anspruch 1, wobei die Säugetier-Blutplättchen menschliche Blutplättchen sind.

3. Zusammensetzung nach Anspruch 1 oder 2, wobei das extrazelluläre Kollagenmatrixmaterial extrazelluläres Matrixmaterial von Schweinen, Rindern, Schafen oder Pferden ist.

4. Zusammensetzung nach einem der vorhergehenden Ansprüche, wobei der bioaktive Anteil ein bioaktiver Anteil eines menschlichen, von Blut abgeleiteten Blutplättchenkonzentrates ist, wobei das Blutplättchenkonzentrat menschliche Blutplättchen und menschliches Plasma enthält, wobei der bioaktive Anteil native Komponenten des Blutplättchenkonzentrates umfasst, beinhaltend Fibrinogen, Albumin, Globulin, TGF-β1, EGF, basic-FGF, PDGF-AA, PDGF-BB, SDF-1α und VEGF.

5. Zusammensetzung nach einem der vorhergehenden Ansprüche, wobei das Fibrinogen des bioaktiven Anteils mit einem Spiegel von unter 20 000 ng/mL vorliegt.

6. Zusammensetzung nach einem der vorhergehenden Ansprüche, wobei der bioaktive Anteil ebenso mindestens eines von und vorzugsweise sämtliche von Plättchen-nativem IL-lb, IL-6, IL-8, IL-10, IL-13, IL-17, IFN-gamma und TNF-alpha beinhaltet.

7. Zusammensetzung nach einem der vorhergehenden Ansprüche, wobei der bioaktive Anteil ein flüssiger bioaktiver Anteil ist, und wobei die Zusammensetzung Folgendes beinhaltet:
ungefähr 0,5 bis 2,5 g/dL Globuline, vorzugsweise ungefähr 1 bis 2 g/dL Globuline;
ungefähr 2 bis 5 g/dL Albumin, vorzugsweise ungefähr 3 bis 4 g/dL Albumin;
ungefähr 100 bis 200 mmol/L Natrium, vorzugsweise ungefähr 120 bis ungefähr 160 mmol/L Natrium;
ungefähr 50 bis 120 mg/dL Triglyceride, vorzugsweise ungefähr 60 bis 110 mg/dL Triglyceride; und/oder
ungefähr 150 bis 300 mg/dL Glukose, vorzugsweise ungefähr 150 bis 250 mg/dL Glukose.

8. Zusammensetzung nach einem der vorhergehenden Ansprüche, wobei der bioaktive Anteil ein flüssiger bioaktiver Anteil ist, und wobei der bioaktive Anteil folgende von den Blutplättchen abgeleitete Komponenten beinhaltet:
Fibrinogen mit einem Spiegel von unter 20 000 ng/ml des flüssigen bioaktiven Anteils;
Albumin mit einem Spiegel von mindestens 2 mg/dL des flüssigen bioaktiven Anteils;
Globulin mit einem Spiegel von mindestens 1 g/dL des flüssigen bioaktiven Anteils;
TGF-β1 mit einem Spiegel von mindestens 5000 pg/mL des flüssigen bioaktiven Anteils;
EGF mit einem Spiegel von mindestens 20 pg/mL des flüssigen bioaktiven Anteils;
FGF-beta mit einem Spiegel von mindestens 5 pg/mL des flüssigen bioaktiven Anteils;
PDGF-AA mit einem Spiegel von mindestens 200 pg/mL des flüssigen bioaktiven Anteils;
PDGF-BB mit einem Spiegel von mindestens 50 pg/mL des flüssigen bioaktiven Anteils;
SDF-1α mit einem Spiegel von mindestens 100 pg/mL des flüssigen bioaktiven Anteils; und
VEGF mit einem Spiegel von mindestens 10 pg/mL des flüssigen bioaktiven Anteils.

9. Verfahren zum Vorbereiten einer bioaktiven Zusammensetzung, Folgendes umfassend:
Auftragen eines bioaktiven Anteils von Säugetier-Blutplättchen auf ein extrazelluläres Kollagenmatrixmaterial, wobei das extrazelluläre Kollagenmatrixmaterial Submukosa umfasst, wobei das extrazelluläre Kollagenmatrixmaterial (ECM) zurückgehaltene sulfatierte, native Glykosaminoglykane eines Quellgewebes für das extrazelluläre Kollagenmatrixmaterial mit einem Spiegel von mindestens ungefähr 500 µg pro Gramm des ECM-Kollagenmaterials auf einer Trockengewichtsgrundlage beinhaltet; und wobei der bioaktive Anteil TGF-β1, EGF, FGF-basic, PDGF-AA, PDGF-BB, SDF-1α und VEGF beinhaltet.

10. Verfahren nach Anspruch 9, wobei die Säugetier-Blutplättchen menschliche Blutplättchen sind.

11. Verfahren nach Anspruch 9 oder 10, wobei das extrazelluläre Kollagenmatrixmaterial extrazelluläres Matrixmaterial von Schweinen, Rindern, Schafen oder Pferden ist.

12. Verfahren nach einem der Ansprüche 9 bis 11, wobei der bioaktive Anteil ein bioaktiver Anteil eines menschlichen, von Blut abgeleiteten Blutplättchenkonzentrates ist, wobei das Blutplättchenkonzentrat menschliche Blutplättchen und menschliches Plasma enthält, wobei der bioaktive Anteil native Komponenten des Blutplättchenkonzentrates umfasst, beinhaltend Fibrinogen, Albumin, Globulin, TGF-β1, EGF, basic-FGF, PDGF-AA, PDGF-BB, SDF-1α und VEGF.

13. Verfahren nach einem der Ansprüche 9 bis 12, wobei das Fibrinogen des bioaktiven Anteils mit einem Spiegel von unter 20 000 ng/mL vorliegt.

14. Verfahren nach einem der Ansprüche 9 bis 13, wobei der bioaktive Anteil ebenso mindestens eines von und vorzugsweise sämtliche von Plättchen-nativem IL-lb, IL-6, IL-8, IL-10, IL-13, IL-17, IFN-gamma und TNF-alpha beinhaltet.

15. Verfahren nach einem der Ansprüche 9 bis 14, wobei der bioaktive Anteil ein flüssiger bioaktiver Anteil ist, und wobei der bioaktive Anteil folgende von den Blutplättchen abgeleitete Komponenten beinhaltet:
Fibrinogen mit einem Spiegel von unter 20 000 ng/ml des flüssigen bioaktiven Anteils;
Albumin mit einem Spiegel von mindestens 2 mg/dL des flüssigen bioaktiven Anteils;
Globulin mit einem Spiegel von mindestens 1 g/dL des flüssigen bioaktiven Anteils;
TGF-β1 mit einem Spiegel von mindestens 5 000 pg/mL des flüssigen bioaktiven Anteils;
EGF mit einem Spiegel von mindestens 20 pg/mL des flüssigen bioaktiven Anteils;
FGF-beta mit einem Spiegel von mindestens 5 pg/mL des flüssigen bioaktiven Anteils;
PDGF-AA mit einem Spiegel von mindestens 200 pg/mL des flüssigen bioaktiven Anteils;
PDGF-BB mit einem Spiegel von mindestens 50 pg/mL des flüssigen bioaktiven Anteils;
SDF-1α mit einem Spiegel von mindestens 100 pg/mL des flüssigen bioaktiven Anteils; und
VEGF mit einem Spiegel von mindestens 10 pg/mL des flüssigen bioaktiven Anteils.

16. Verfahren nach einem der Ansprüche 9 bis 15, ebenso umfassend Trocknen des extrazellulären Kollagenmatrixmaterials nach dem Auftragen und vorzugsweise wobei das Trocknen Lyophilisieren umfasst.

17. Verfahren nach einem der Ansprüche 9 bis 16, zudem umfassend Spülen des extrazellulären Kollagenmatrixmaterials nach dem Auftragen, zum Entfernen eines Teils des bioaktiven Anteils von dem extrazellulären Kollagenmatrixmaterial.

18. Verfahren nach Anspruch 17, wobei der Teil eine Menge von mindestens einem Wachstumsfaktor, und vorzugsweise eine Vielzahl von Wachstumsfaktoren beinhaltet.

19. Verfahren nach Anspruch 17 oder 18, ebenso umfassend Trocknen des extrazellulären Kollagenmatrixmaterials nach dem Spülen und vorzugsweise wobei das Trocknen Lyophilisieren umfasst.

20. Zusammensetzung oder Verfahren nach einem der vorhergehenden Ansprüche, wobei das extrazelluläre Kollagenmatrixmaterial Kollagen in einer Menge von mindestens ungefähr 80 Gewichtsprozent auf einer Trockengewichtsbasis umfasst.

21. Zusammensetzung oder Verfahren nach einem der vorhergehenden Ansprüche, wobei das extrazelluläre Kollagenmatrixmaterial (ECM) zurückgehaltene sulfatierte, native Glykosaminoglykane eines Quellgewebes für das extrazelluläre Kollagenmatrixmaterial mit einem Spiegel von mindestens ungefähr 1 000 µg pro Gramm des ECM-Kollagenmaterials auf einer Trockengewichtsbasis beinhaltet.

22. Zusammensetzung oder Verfahren nach einem der vorhergehenden Ansprüche, wobei die Submukosa Darm-, Harnblasen- oder Magensubmukosa ist.

23. Set zum Vorbereiten einer Zusammensetzung, umfassend ein extrazelluläres Kollagenmatrixmaterial (ECM) nach einem der Ansprüche 1 bis 22 und einen bioaktiven Anteil aus Säugetier-Blutplättchen nach einem der Ansprüche 1 bis 22.

## Revendications

1. Composition comprenant :
un matériau de matrice extracellulaire collagène, dans laquelle le matériau de matrice extracellulaire collagène comprend de la sous-muqueuse, et dans laquelle le matériau de matrice extracellulaire (ECM) collagène inclut des glycosaminoglycanes sulfatés retenus qui sont originaires d'un tissu source pour le matériau de matrice extracellulaire collagène à un niveau d'au moins environ 500 µg par gramme du matériau d'ECM collagène sur une base poids à sec ; et
une fraction bioactive de plaquettes de mammifère qui est appliquée sur le matériau de matrice extracellulaire collagène, dans laquelle la fraction bioactive inclut du TGF-β1, de l'EGF, du FGF basique, du PDGF-AA, du PDGF-BB, du SDF-1α et du VEGF.

2. Composition selon la revendication 1, dans laquelle les plaquettes de mammifère sont des plaquettes d'être humain.

3. Composition selon la revendication 1 ou 2, dans laquelle le matériau de matrice extracellulaire collagène est un matériau de matrice extracellulaire porcine, bovine, ovine ou équine.

4. Composition selon l'une quelconque des revendications précédentes, dans laquelle la fraction bioactive est une fraction bioactive d'un concentré de plaquettes dérivées du sang humain, le concentré de plaquettes contenant des plaquettes de l'être humain et du plasma de l'être humain, la fraction bioactive comprenant des composants originaires du concentré de plaquettes incluant du fibrinogène, de l'albumine, de la globuline, du TGF-β1, de l'EGF, du FGF basique, du PDGF-AA, du PDGF-BB, du SDF-1α et du VEGF.

5. Composition selon l'une quelconque des revendications précédentes, dans laquelle le fibrinogène de la fraction bioactive est présent à un niveau qui est inférieur à 20 000 ng/mL.

6. Composition selon l'une quelconque des revendications précédentes, dans laquelle la fraction bioactive inclut également au moins l'un de, et de préférence chacun de IL-lb, IL-6, IL-8, IL-10, IL-13, IL-17, IFN-gamma et TNF-alpha qui sont originaires des plaquettes.

7. Composition selon l'une quelconque des revendications précédentes, dans laquelle la fraction bioactive est une fraction bioactive liquide, et dans laquelle la composition inclut :
environ 0,5 à 2,5 g/dL de globulines, de préférence environ 1 à 2 g/dL de globulines ;
environ 2 à 5 g/dL d'albumine, de préférence environ 3 à 4 g/dL d'albumine ;
environ 100 à 200 mmol/L de sodium, de préférence environ 120 à environ 160 mmol/L de sodium ;
environ 50 à 120 mg/dL de triglycérides, de préférence environ 60 à 110 mg/dL de triglycérides ; et/ou
environ 150 à 300 mg/dL de glucose, de préférence environ 150 à 250 mg/dL de glucose.

8. Composition selon l'une quelconque des revendications qui précédentes, dans laquelle la fraction bioactive est une fraction bioactive liquide, et dans laquelle la fraction bioactive inclut les composants suivants qui sont dérivés à partir des plaquettes :
du fibrinogène à un niveau inférieur à 20 000 ng/ml de la fraction bioactive liquide ;
de l'albumine à un niveau d'au moins 2 mg/dL de la fraction bioactive liquide ;
de la globuline à un niveau d'au moins 1 g/dL de la fraction bioactive liquide ;
du TGF-β1 à un niveau d'au moins 5000 pg/mL de la fraction bioactive liquide ;
de l'EGF à un niveau d'au moins 20 pg/mL de la fraction bioactive liquide ;
du FGF-beta à un niveau d'au moins 5 pg/mL de la fraction bioactive liquide ;
du PDGF-AA à un niveau d'au moins 200 pg/mL de la fraction bioactive liquide ;
du PDGF-BB à un niveau d'au moins 50 pg/mL de la fraction bioactive liquide ;
du SDF-la à un niveau d'au moins 100 pg/mL de la fraction bioactive liquide ; et
du VEGF à un niveau d'au moins 10 pg/mL de la fraction bioactive liquide.

9. Procédé pour préparer une composition bioactive, comprenant :
l'application d'une fraction bioactive de plaquettes de mammifère sur un matériau de matrice extracellulaire collagène, dans lequel le matériau de matrice extracellulaire collagène comprend de la sous-muqueuse, dans lequel le matériau de matrice extracellulaire (ECM) collagène inclut des glycosaminoglycanes sulfatés retenus qui sont originaires d'un tissu source pour le matériau de matrice extracellulaire collagène à un niveau d'au moins environ 500 µg par gramme du matériau d'ECM collagène sur une base poids à sec et dans lequel la fraction bioactive inclut du TGF-β1, de l'EGF, du FGF basique, du PDGF-AA, du PDGF-BB, du SDF-1α et du VEGF.

10. Procédé selon la revendication 9, dans lequel les plaquettes de mammifère sont des plaquettes d'être humain.

11. Procédé selon la revendication 9 ou 10, dans lequel le matériau de matrice extracellulaire collagène est un matériau de matrice extracellulaire porcine, bovine, ovine ou équine.

12. Procédé selon l'une quelconque des revendications 9 à 11, dans lequel la fraction bioactive est une fraction bioactive d'un concentré de plaquettes dérivées du sang humain, le concentré de plaquettes contenant des plaquettes de l'être humain et du plasma de l'être humain, la fraction bioactive comprenant des composants originaires du concentré de plaquettes incluant du fibrinogène, de l'albumine, de la globuline, du TGF-β1, de l'EGF, du FGF basique, du PDGF-AA, du PDGF-BB, du SDF-1α et du VEGF.

13. Procédé selon l'une quelconque des revendications 9 à 12, dans lequel le fibrinogène de la fraction bioactive est présent à un niveau qui est inférieur à 20 000 ng/mL.

14. Procédé selon l'une quelconque des revendications 9 à 13, dans lequel la fraction bioactive inclut également au moins l'un de, et de préférence chacun de IL-lb, IL-6, IL-8, IL-10, IL-13, IL-17, IFN-gamma et TNF-alpha qui sont originaires des plaquettes.

15. Procédé selon l'une quelconque des revendications 9 à 14, dans lequel la fraction bioactive est une fraction bioactive liquide, et dans lequel la fraction bioactive inclut les composants suivants qui sont dérivés à partir des plaquettes :
du fibrinogène à un niveau inférieur à 20 000 ng/ml de la fraction bioactive liquide ;
de l'albumine à un niveau d'au moins 2 mg/dL de la fraction bioactive liquide ;
de la globuline à un niveau d'au moins 1 g/dL de la fraction bioactive liquide ;
du TGF-β1 à un niveau d'au moins 5000 pg/mL de la fraction bioactive liquide ;
de l'EGF à un niveau d'au moins 20 pg/mL de la fraction bioactive liquide ;
du FGF-beta à un niveau d'au moins 5 pg/mL de la fraction bioactive liquide ;
du PDGF-AA à un niveau d'au moins 200 pg/mL de la fraction bioactive liquide ;
du PDGF-BB à un niveau d'au moins 50 pg/mL de la fraction bioactive liquide ;
du SDF-1α à un niveau d'au moins 100 pg/mL de la fraction bioactive liquide ; et
du VEGF à un niveau d'au moins 10 pg/mL de la fraction bioactive liquide.

16. Procédé selon l'une quelconque des revendications 9 à 15, comprenant également le séchage du matériau de matrice extracellulaire collagène après ladite application et de préférence, dans lequel le séchage comprend une lyophilisation.

17. Procédé selon l'une quelconque des revendications 9 à 16, comprenant également le rinçage de la matrice extracellulaire collagène après ladite application pour supprimer une partie de la fraction bioactive du matériau de matrice extracellulaire collagène.

18. Procédé selon la revendication 17, dans lequel la partie inclut une quantité d'au moins un facteur de croissance et de préférence, une pluralité de facteurs de croissance.

19. Procédé selon la revendication 17 ou 18, comprenant également le séchage du matériau de matrice extracellulaire collagène après ledit rinçage et de préférence, dans lequel le séchage comprend une lyophilisation.

20. Composition ou procédé selon l'une quelconque des revendications précédentes, dans laquelle ou lequel le matériau de matrice extracellulaire collagène inclut un collagène à une quantité d'au moins environ 80 % en poids sur une base poids à sec.

21. Composition ou procédé selon l'une quelconque des revendications précédentes, dans laquelle ou lequel le matériau de matrice extracellulaire (ECM) collagène inclut des glycosaminoglycanes sulfatés retenus qui sont originaires d'un tissu source pour le matériau de matrice extracellulaire collagène à un niveau d'au moins environ 1000 µg par gramme du matériau d'ECM collagène sur une base poids à sec.

22. Composition ou procédé selon l'une quelconque des revendications précédentes, dans laquelle ou lequel la sous-muqueuse est de la sous-muqueuse intestinale, de la vessie ou de l'estomac.

23. Kit pour préparer une composition, comprenant un matériau de matrice extracellulaire (ECM) collagène tel que défini selon l'une quelconque des revendications 1 à 22, et une fraction bioactive de plaquettes de mammifère telle que définie selon l'une quelconque des revendications 1 à 22.
